Europäisches Patentamt

European Patent Office

Office européen des brevets

(11) Publication number: **0 223 156 B1**

# EUROPEAN PATENT SPECIFICATION

(45) Date of publication of patent specification: 22.05.91    (51) Int. Cl.⁵: **C12N  15/64**, C12N 15/70

(21) Application number: **86115366.6**

(22) Date of filing: **06.11.86**

(54) Streptomycin sensitive gene having a lot of cloning sites, plasmid vector containing the same and microorganism containing the vector.

(30) Priority: **12.11.85 JP 253192/85**
      **14.08.86 JP 190798/86**

(43) Date of publication of application:
      **27.05.87 Bulletin  87/22**

(45) Publication of the grant of the patent:
      **22.05.91 Bulletin  91/21**

(84) Designated Contracting States:
      **AT BE CH DE ES FR GB GR IT LI LU NL SE**

(56) References cited:

      **GENE, vol. 15, 1981 (Amsterdam) D. DEAN "A plasmid cloning vector for the direct selection of strains carrying recombinant plasmids" pages 99-102**

      **GENE, vol. 27, no. 1, January 1984 (Amsterdam) G. BRADY et al. "New cosmid vectors developed for eukaryotic DNA cloning" pages 223-232**

(73) Proprietor: **HOECHST JAPAN LIMITED**
      **10-16, 8-chome, Akasaka, Minato-ku**
      **Tokyo(JP)**

(72) Inventor: **Iwaki née Kume, Akiko**
      **Tanaka Blg. 302 2-4-24 Minamidai**
      **Kawagoe-Shi Saitama-Ken(JP)**
      Inventor: **Iwasaki, Wakako**
      **Dohkan-Haitsu 405 3-9-1 Kamiigusa**
      **Suginami-Ku**
      **Tokyo(JP)**
      Inventor: **Toba, Mari**
      **1-37-23, Kishi-Machi**
      **Kawagoe-Shi Saitama-Ken(JP)**
      Inventor: **Takeshita, Sunao**
      **Nippon Hoechst Shainryo 3-7-22, Minamidai**
      **Kawagoe-Shi Saitama-Ken(JP)**
      Inventor: **Hashimoto, Tamotsu**
      **1-5-10-309, Sakae-Cho**
      **Asaka-Shi Saitama-Ken(JP)**
      Inventor: **Okazaki, Hiroshi**
      **3-9-11-402, Irima-Cho**
      **Chofu-Shi Tokyo(JP)**
      Inventor: **Fukuda, Akio**
      **3-12-16 Nakameguro Meguro-Ku**
      **Tokyo(JP)**

(74) Representative: **Klein, Otto, Dr. et al**
      **Hoechst AG Zentrale Patentabteilung Postfach 80 03 20**
      **W-6230 Frankfurt am Main 80(DE)**

## Description

### Background of the Invention

This invention relates to a streptomycin sensitive gene (strA+ or Sms) having many cloning sites and a plasmid vector containing the same. Also, the present invention relates to a microorganism containing the plasmid vector.

In genetic manipulation, the technique of cloning the whole of a desired gene or subcloning a part of the gene is an important technique which is most frequently used. During this cloning (including subcloning), investigation of various properties possessed by the plasmid vector used, specifically size, number of copies per cell, kinds and number of the cloning sites, kinds of marker genes such as drug resistance, etc., for selection and dependency on the genetic properties on the side of the host, for the purpose of selecting how to use it or how it is to be combined, can be a great factor which influences efficiency as well as success or failure of the experiment. The methods for selecting efficiently on a medium plate only the vector having particularly a foreign DNA fragment incorporated among these properties of plasmid vector may be mainly classified into the negative selection method and the positive selection method.

In the negative selection method, a foreign DNA fragment is incorporated at the cloning site in the region coding for a specific drug resistant gene on the plasmid vector, and the property of a host transformed by the recombinant plasmid, that it can no longer grow on a medium containing the drug through the change of the phenotype from resistant to sensitive to the drug is utilized. On the other hand, in the positive selection method, the property such that only the host having the plasmid vector incorporated with a foreign DNA fragment can grow on a specific selective medium or that only the microorganism cells having the desired recombinant product among the colonies grown can be easily discriminated by the change in tone, etc., has been utilized.

When these two methods are compared with each other, the latter positive selective method requiring only one step selection is more efficient than the negative selection which requires two steps of selection.

At present, a typical example of the former of the plasmid vectors frequently used is pBR322 having both of the resistant genes to the antibiotics of ampicillin and tetracycline, and also there are a number of plasmid vectors which have been improved based on the pBR322, and further have drug resistant genes resistant to kanamycin or chloramphenicol incorporated therein. On the other hand, as an example of the latter, only pUC type plasmid vectors in which only colonies having the plasmid incorporated with a foreign DNA fragment change from blue to white have been used widely, and their kinds are still limited.

For this reason, it is greatly desired to have a new plasmid vector capable of positive selection which can be used easily.

As a plasmid vector capable of positive selection, a plasmid vector designated as pNO1523 containing a gene having sensitivity to streptomycin (called Sms, strA+ or rpsL+) has been already prepared [Gene, Vol. 15, pp 99 ~ 102 (1981)]. This is presently sold from Pharmacia, Inc. in Sweden and is available to everybody.

The pNO1523 contains both of the gene resistant to ampicillin (ApR) and the gene coding for the S12 ribosome protein which is streptomycin sensitive as the markers for selection. When a host resistant to streptomycin controlled by the rpsL gene such as the HB101 strain derived from Escherichia coli K12 strain which is one of the hosts frequently used in recombinant DNA experiments is transformed by pNO1523, the transformant becomes sensitive to streptomycin and can no longer grow on a selective medium containing streptomycin. However, when a foreign DNA fragment is incorporated at the cloning site existing within the streptomycin sensitive gene, the streptomycin sensitive gene is destroyed and the transformed strain becomes resistant to streptomycin. When ampicillin is also added in the selective medium, the host itself and the transformant with the pNO1523 cannot grow, but only the transformant with the recombinant product of pNO1523 incorporated with a foreign DNA fragment can grow in the medium.

In spite of having such useful properties, the pNO1523 has the drawback that it has a few recognition sites of restriction enzymes which can be utilized as the cloning site of a foreign DNA. More specifically, although the streptomycin sensitive gene of pNO1523 has recognition sites of five restriction enzymes of Hpal, Pvull, Sphl, Pstl and Smal, the three of Pvull, Sphl and Pstl among them exist at the outside portion of said gene and therefore it is difficult to utilize these as the cloning sites. Also, since the two cloning sites of Hpal and Smal are each blunt end, efficiency of cloning is low and therefore this plasmid vector can find only very limited uses.

## SUMMARY OF THE INVENTION

The present inventors have found that a new streptomycin sensitive gene can be obtained in which a lot of recognition sites of restriction enzymes have been introduced into the streptomycin sensitive gene of pNO1523 having the advantage of being capable of positive selection by changing partially the base sequence of the streptomycin sensitive gene without changing the characteristics of the streptomycin sensitivity.

The present invention is based on such a finding, and it concerns the streptomycin sensitive gene itself, a plasmid vector containing the same and a microorganism containing the vector.

More specifically, the streptomycin sensitive gene according to the present invention has at least one group of recognition sites selected from the groups as set forth below in the three senses as mentioned above:

a group of restriction enzyme recognition sites consisting of AsuII, SacI, BalI and XhoI (hereinafter called No. 1 group of recognition sites);

a group of restriction enzyme recognition sites consisting of MluI, SnaI, EcoRI, MstI and BstEII (hereinafter called No. 2 group of recognition sites);

a group of restriction enzyme recognition sites consisting of BclI, NotI and BglII (hereafter called No. 3 group of recognition sites); and

a group of restriction enzyme recognition sites consisting of KpnI, SacII, XbaI, SalI, ClaI, PvuI and StuI (hereinafter called No. 4 group of recognition sites).

The NotI site included in the No. 3 group of recognition sites is also a recognition site by XmaIII, and each of the groups of recognition sites is cleaved with at least four restriction enzymes.

Into these groups of recognition sites, it is also possible to introduce one or more recognition sites of another restriction enzymes.

For example, between MstI and BstEII of the No. 2 group of recognition sites, HindIII site can be introduced, and also adjacent to (upstream of) KpnI of the No. 4 group recognition sites, BamHI site can be introduced. The present invention is also inclusive of a gene containing additionally such a recognition site of another restriction enzyme as well as a plasmid containing the same and a microorganism containing the plasmid.

The DNA coding for the streptomycin sensitive gene obtained by the present invention has a lot of recognition sites by restriction enzymes. And, when a foreign DNA is incorporated into the recognition sites, it has the property that the streptomycin sensitive gene is destroyed. For this reason, various kinds of foreign DNA can be easily incorporated into the abundant recognition sites of restriction enzymes. The recombinant DNA having a foreign gene incorporated therein loses the streptomycin sensitivity and has the property of growing in a medium in which streptomycin is added, and therefore it can be easily selected by utilizing this property. Accordingly, a plasmid vector which is very useful for genetic recombinant manipulation can be provided by the present invention.

According to the Examples of the present invention as described below, (a) pHSG665 in which only No. 1 group of recognition sites is introduced, (b) p1626 in which No. 1 and No. 4 groups of recognition sites are introduced, (c) p1630 in which No. 1, No. 3 and No. 4 groups of recognition sites are introduced, (d) pHSG666 in which, in addition to No. 1, No. 3 and No. 4 groups of recognition sites, the recognition site of BamHI is further introduced adjacent to (upstream of) KpnI in No. 4 group of recognition sites, (e) pHSG671 in which No. 1, No.2, No. 3 and No. 4 groups of recognition sites and the recognition site of BamHI are introduced and (f) pHSG670 in which, in addition to No. 1, No.2, No.3 and No. 4 groups of recognition sites and the recognition site of BamHI, the site of HindIII is introduced between EcoRI and BstEII in No. 2 group of recognition sites are produced as examples of the plasmid vector of the present invention having the streptomycin sensitive gene (the present invention is not limited by these examples, but, for example, one in which only one group of recognition sites of No. 1, No. 2, No. 3 and No. 4 groups of recognition sites are introduced or those containing any desired two or three of the groups of recognition sites are included in the present invention. It is also possible that the recognition site of a restriction enzyme not referred to in the present invention can be additionally contained).

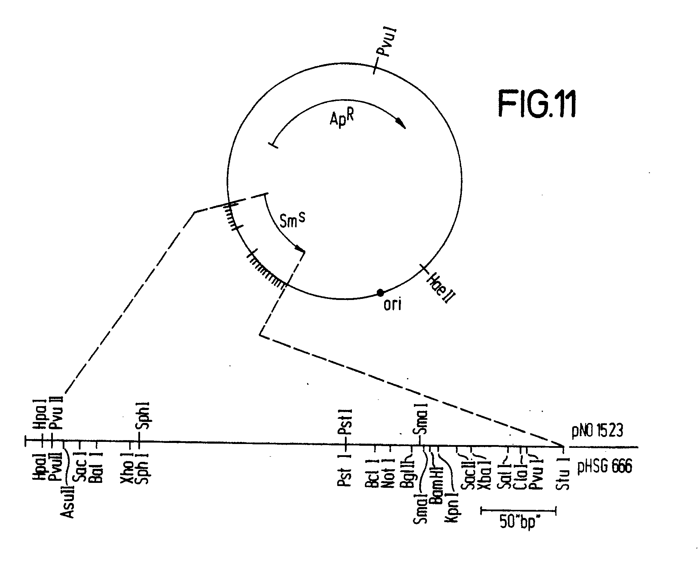

Of the plasmid vectors obtained according to the present invention, the most important is pHSG666 and its restriction enzyme map is shown in Fig. 11. It has a streptomycin sensitive gene having No. 1, No. 3 and No. 4 groups of recognition sites and the recognition site of BamHI, and also has an ampicillin resistant gene as another selection marker and a replicon derived from pUC9 and its characteristics are as follows.

① The plasmid has a size which is about half as compared with pNO1523. For this reason, it becomes easier to incorporate a greater DNA fragment.

② The number of copies within the cell is increased as compared with pNO1523, and therefore a plasmid DNA sufficient in amount for cloning, for example, about 200 μg even from a small amounts of a culture broth of about 100 ml can be easily purified.

③ In order to utilize the recognition sites of the restriction enzymes at the three positions of PvuII, SphI and PstI inherently contained in the streptomycin sensitive gene of pNO1523 as the cloning sites, the recognition sites of these three kinds of restriction enzymes existing on the outside of said gene are deleted, and therefore the SphI and PstI sites having cohesive ends readily handleable in cloning became utilizable. Also, since the SphI and PstI sites became utilizable, they can be used also for cloning of the cDNA fragment modified at the ends by the GC polymer attachment method.

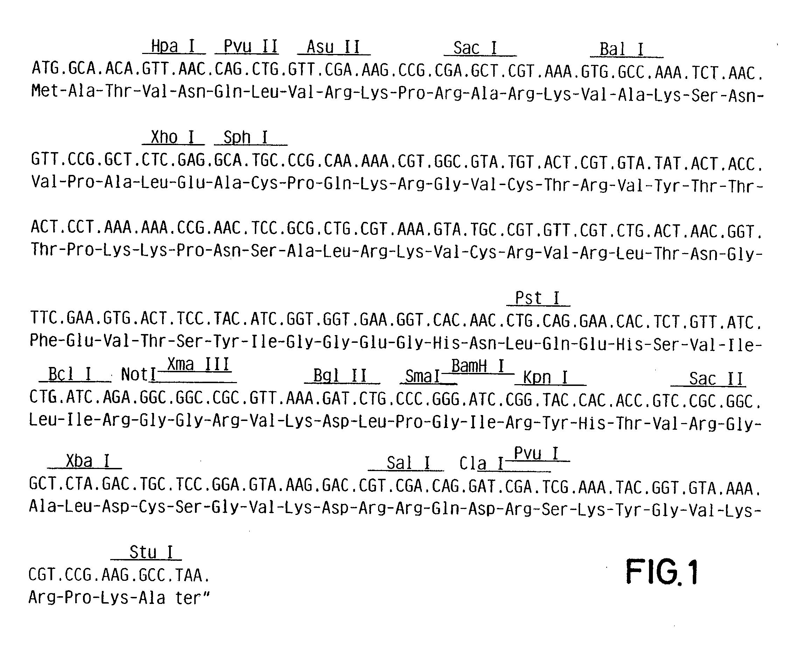

④ Other than the above ③, into the improved streptomycin sensitive gene reconstructed with the synthetic DNA fragment, the following 12 kinds of cloning sites as shown in Fig. 1, namely AsuII, SacI, BalI, XhoI, BclI, NotI (XmaIII), BglII, KpnI, SacII, XbaI, PvuI and StuI were newly added by only changing the bases without changing the amino acid residues, whereby these sites were rendered utilizable.

⑤ In addition to the above ③ and ④, further into the improved type streptomycin sensitive gene reconstructed with the synthetic DNA fragment, the cloning sites at the three positions of BamHI, SalI and ClaI are added by substituting the amino acid residues respectively without impairing the function of the streptomycin sensitive gene, whereby these sites were rendered utilizable.

Thus, the improved type streptomycin sensitive plasmid vector, having the above characteristics ① ~ 5 accomplished by the present invention and also having the effect as confirmed by the experimental examples shown below, provides a means for efficient cloning in recombinant DNA experiments.

The microorganism to be used as the host in the present invention is typically Escherichia coli, and the Escherichia coli K-12 strain corresponding to the B1 level of biological containment as determined in "Recombinant DNA Experimental Guidelines" (edited by Agency of Science and Technology, revised on August 31, 1982) and various kinds of strains derived therefrom can be used. A typical example of such strains is HB101 strain. This strain has been utilized most frequently in recombinant DNA experiments by researchers in both in our country and abroad and is readily available, [for example, commercially sold from Bethesda Research Laboratory, Maryland, U.S.A. (code number: BRL #8260SA) and available readily to everybody]. Also, transformation of such a host with said plasmid vector can be practiced according to the calcium treatment method [Journal of Molecular Biology (J. Mol. Biol.), Vol. 53, pp. 159 ~ 162 (1970)] which have been generally widely practiced. Thus, one of the advantages of the present invention resides in that a transformant can be obtained with relative ease with the use of a strain of Escherichia coli such as HB101, etc., which can be easily used.

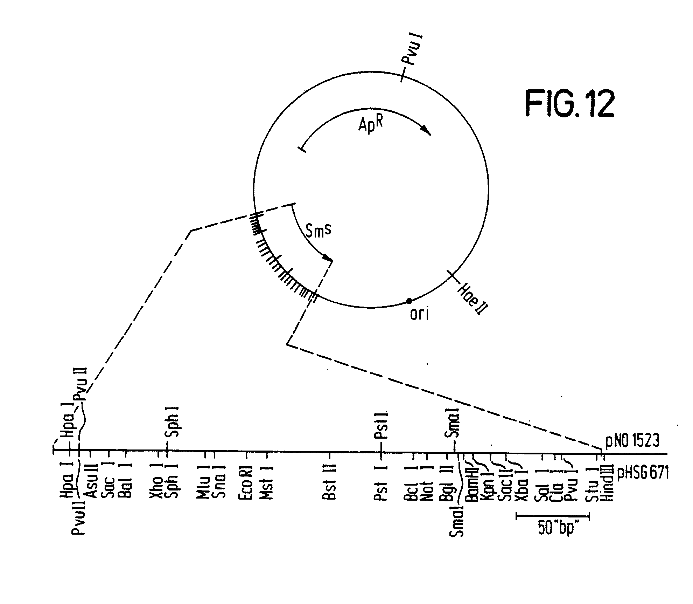

Of the plasmid vectors according to the present invention, another most important one is pHSG671. This vector also has substantially the same advantages (a) to (e) as described above. Moreover pHSG671 has further new recognition sites of MluI, SnaI, EcoRI, MstI and BstEII. Its restriction enzyme cleavage map is shown in Fig. 12.

## DETAILED DESCRIPTION OF THE INVENTION

The present invention provides an improved type streptomycin sensitive gene having many cloning sites added thereto and a recombinant plasmid vector containing the same capable of positive selection.

Concerning the so called DNA recombinant manipulation comprising excising, purifying, combining DNA fragments, determining the base sequences thereof, etc., some textbooks have been already known and therefore reference should be made to those literatures about the necessary informations except for the detailed description of the present invention as given below. To mention some of the suitable references, there may be, for example, ① "Molecular Cloning, A Laboratory Manual" by Dr. Maniatis, et al., published by Cold Spring Harbor Laboratory, U.S.A., 1982 and ② "Genetic Manipulation Experimental Method", edited by Prof. Yasutaka Takagi, Kodansha, 1980.

Also, concerning the methods of synthesis and purification of DNA fragments which are important steps in preparation of the improved type plasmid vector in the present invention, other than the detailed description of the present invention given below, reference can be made to, for example, ③ "User Bulletin", Vol. 13, pp. 1 ~ 27, 1984, published by Applied Biosystems, Inc., U.S.A.

The host employed in the present invention was primarily HB101 strain of Escherichia coli K12 (rec⁻, r⁻, m⁻, str$^R$). As the host for the plasmid vectors pUC18 and pUC19, Om214 strain of Escherichia coli (rec⁺, r⁻, m⁻, M15, str$^R$, F⁺) was employed. For all of the bases, restriction enzymes, T₄DNA ligase, T₄DNA polymerase, buffers, etc., all commercial products were employed. All of the operations of digestion, preparation of blunt end by filling in by use of T₄DNA polymerase, linking, transformation by calcium treatment, etc., have been practiced in the present invention following conventional manners, and details about their conditions are not particularly described here.

The present invention concerns streptomycin sensitive genes and their related products, and these are specified by the restriction enzyme recognition sites. Expressions of the restriction enzyme recognition sites referred to and their contents are well known to those skilled in the art.

Summary of the Steps (I)

(1) Preparation of a plasmid vector for performing recombination with ease:

The present inventors, for the purpose of progressing easily a change in base sequence of the streptomycin sensitive gene of pNO1523, newly prepared a plasmid vector of pHSG664 by binding a streptomycin sensitive gene excised from pNO1523 to a DNA fragment coding for an ampicillin resistant gene and a replicon excised from pUC9.

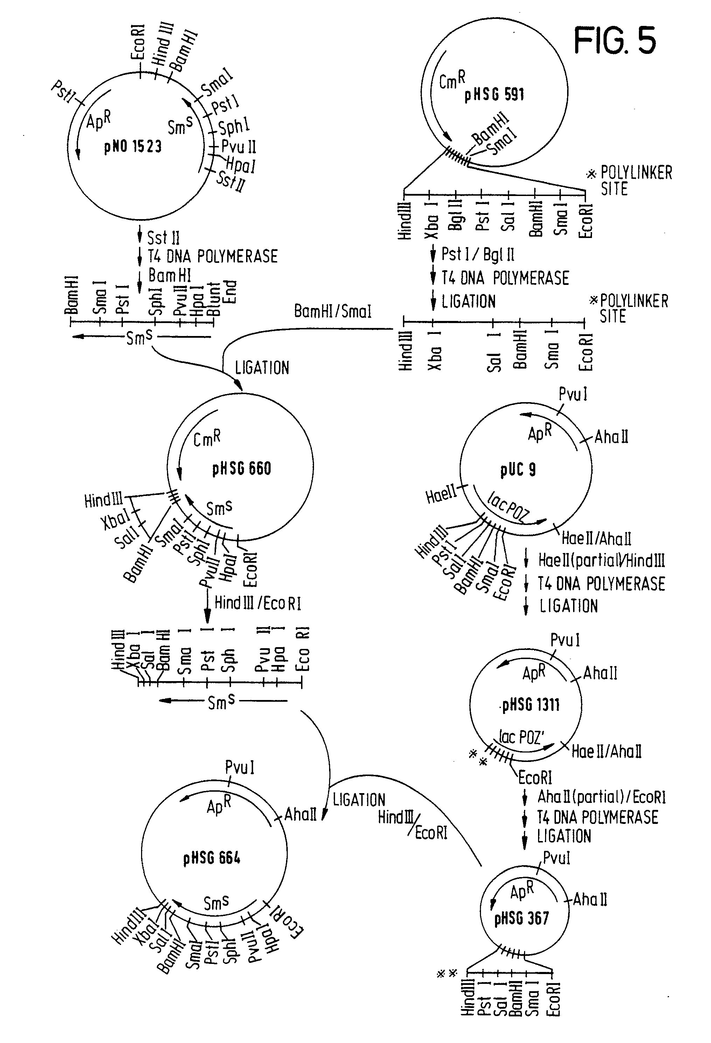

The preparation route of pHSG664 and its restriction enzyme map are shown in Fig. 5.

(2) Programming of synthetic DNA fragment:

For introduction of recognition sites of restriction enzymes by changing partially the base sequence of the streptomycin sensitive gene of pNO1523, recognition sites of restriction enzymes may be made by replacing the bases within the range in which the amino acids encoded are not changed, or alternatively they may be made with a change in the amino acids encoded within the range in which streptomycin sensitivity is not lost. Since change in amino acids will lead to loss of streptomycin sensitivity, it should be made as minimum as possible. Also, it is necessary to avoid formation of a plural number of the recognition sites of the same restriction enzyme as the result of replacement of bases for preparation of the recognition sites of restriction enzymes.

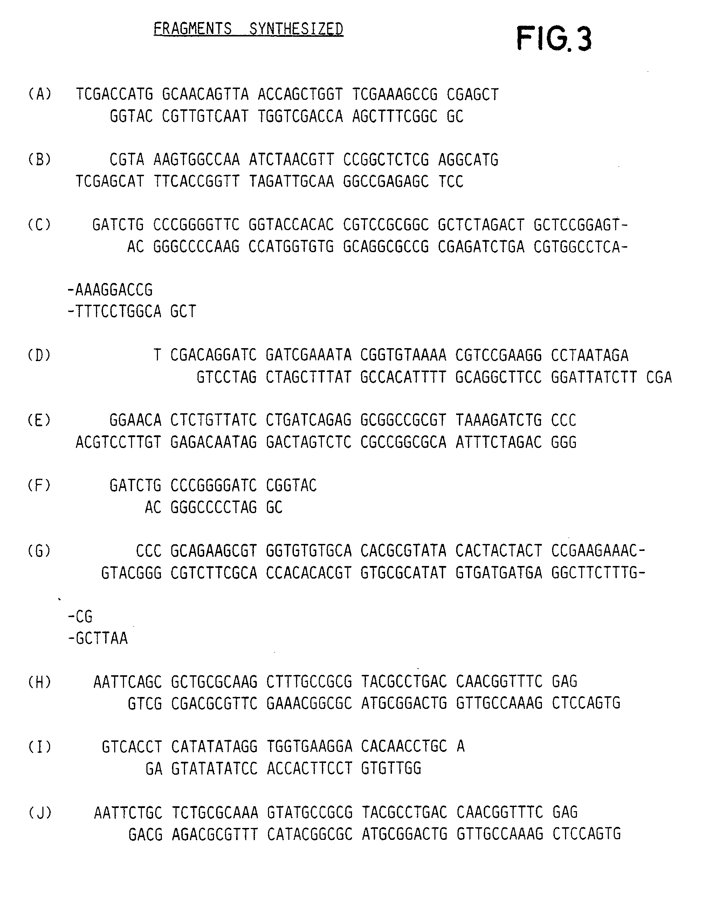

In the present invention, the No. 1 group of recognition sites, namely AsuII, SacI, BalI and XhoI sites, the No. 2 group of recognition sites, namely MluI, SnaI, EcoRI, MstI and BstEII sites, and the No. 3 group of recognition sites, namely BclI, NotI and BglII sites can be introduced by changing only the bases without change of amino acids. Of the No. 4 group of recognition sites, KpnI, SacII, XbaI, PvuI and StuI sites can be also introduced by changing only the bases without change of amino acid residues. Of the No. 4 group recognition sites, SalI and ClaI have base sequences introduced so as to replace the amino acid residues without impairing the function of the streptomycin sensitive gene. More specifically, the 111th lysine residue is replaced with arginine residue and the 113th alanine residue with aspartic acid residue. The HindIII site introduced additionally into pHSG670 is accompanied with change of amino acids, namely the change of the 52th valine residue to leucine residue. The BamHI site introduced additionally into pHSG666 is accompanied with change of amino acids, namely the change of the 93th valine residue to isoleucine residue. The respective base sequences of the synthetic DNA fragments used in the present invention are summarized in Fig. 3.

In some cases, a part of the base sequence has been changed without changing the sequence of amino acid residues for some purpose other than introducing recognition sites of restriction enzymes, and this has been done for preventing formation of a secondary structure in the streptomycin sensitive gene which inhibits leading of said gene by introducing new recognition sites into the gene.

(3) Synthesis and recombination of DNA fragments:

In the present invention, the DNA sequences of a series of recognition sites by restriction enzymes are synthesized according to the phosphoramidite method by a DNA synthesizer (produced by Applied Biosystems, Inc., U.S.A.), purified and then recognition sites by a new restriction enzyme are introduced by replacing a part of the streptomycin sensitive gene derived from pNO1523 with their synthetic DNA fragments. The length of the DNA fragment synthesized should be safely a length of not more than 65 bases at the longest. When the base sequence having a group of recognition sites is long, it is desirable to synthesize DNA fragments divided into two or more fragments and binding them according to the genetic engineering method to form a base sequence having a series of recognition sites before recombination with the streptomycin sensitive gene derived from pNO1523. In the present invention, the DNA fragment corresponding to the No. 1 group of recognition sites (AsuII, SacI, BalI and XhoI) was prepared by binding the two synthetic DNA fragments A and B according to the genetic engineering method. The fragment corresponding to the No. 2 group of recognition sites (MluI, SnaI, EcoRI, MstI and BstEII) was prepared in an Example of the present invention by binding the three synthetic DNA fragments G, H and I according to the genetic engineering method and further by replacing the portion introduced from the fragment H with a synthetic DNA fragment J. This is because pHSG670 having additionally Hind III site was produced in advance by means of fragment H containing additionally Hind III site. However, it is ordinarily prepared by combining the three synthetic DNA fragments G, J, and I according to the genetic engineering method. As for the DNA fragment E corresponding to the No. 3 group of recognition fragments (BclII, NotI and BglII), its whole fragment was synthesized. The DNA fragment corresponding to the No. 4 group of recognition sites (KpnI, SacII, XbaI, SalI, ClaI, PvuI and StuI) was prepared by binding the two synthetic DNA fragments C and D according to the genetic engineering method. The synthetic DNA fragment F is for introducing an additional site BamHI adjacent to (upstream of) KpnI of the No. 4 group of recognition sites.

Synthetic DNA fragments having recognition sites of a restriction enzyme required for recombination thereof with the streptomycin sensitive DNA derived from pNO1523 are added, if desired, in addition to the DNA corresponding to the group of recognition sites newly introduced. Introduction of a synthetic DNA fragment into the streptomycin sensitive gene derived from pNO1523 may be practiced in a conventional manner in which the site of pNO1523 to be replaced is excised with a restriction enzyme and the synthetic DNA fragment is inserted therein.

Summary of Steps (II)

(1) Preparation of a plasmid vector pHSG664

The present inventors, in modifying the streptomycin sensitive gene contained in pNO1523, for the purpose of progressing smoothly binding with a synthetic DNA fragment, prepared a new pHSG664 having the streptomycin sensitive gene of pNO1523.

The pHSG664 has the streptomycin sensitive gene of pNO1523, a polylinker portion comprising BamHI, SalI, XbaI and HindIII existing downstream of said gene, EcoRI site existing upstream of said gene, an ampicillin resistant gene of pUC9 and a replicon derived from pUC9.

Preparation of the pHSG664 is described in detail in Fig. 5 and Reference example.

The pHSG664 has the following advantages as compared with the original pNO1523.

    i) The vector size becomes about half, whereby a greater DNA fragment can be incorporated.

    ii) The number of copies within a cell is increased, whereby the plasmid DNA can be purified easily even from a small amount of a culture broth of microorganism cells.

    iii) By deletion of the recognition sites of PvuII, SphI and PstI existing outside of the streptomycin sensitive gene, these three kinds of recognition sites existing within said gene can be used as the cloning sites.

(2) Synthesis and purification of a DNA fragment in which recognition sites of restriction enzyme are introduced:

    i) The length of the DNA fragment prepared in synthesis is made 65 bases at the longest, and a fragment having cloning sites necessary for linking at the both ends is synthesized. The fragment to be used for recombination is a double-strand, and this is prepared by synthesizing and purifying the respective single-strand and subjecting them to annealing.

    ii) The respective DNA fragments are all synthesized by an automatically programmed DNA synthesizer "Applied Biosystems Model 380A" (produced by Applied Biosystems, Inc., U.S.A.). Concerning the DNA synthesizer used in the present invention "Applied Biosystems Model 380A", details of the synthetic reaction, the structure of the machine, the software for operation and synthetic examples are described in detail in the literature of American Biotechnology Laboratory, Vol. 2, pp. 52 ~ 59 (1984).

    iii) Since the respective synthesized DNA fragments are obtained from the DNA synthesizer as acetonitrile solutions of derivatives with protective groups attached onto the respective basis, treatment is carried out with addition of an equal amount of conc. ammonia at $55°C$ for 5 hours to effect deprotection. Further, the solution containing deprotected DNA fragments is concentrated to dryness, which is dissolved in $50 \mu l$ of 80% formamide solution by heating at $95°C$ for several minutes, and the DNA fragments with a desired length are separated and purified in 0.1M tris-borate-EDTA buffer according to vertical electrophoresis by use of a 10% polyacrylamide gel (containing 7M urea; thickness 3 mm, height 40 cm, width 30 cm). Since the DNA fragments can be visualized by irradiation of UV-ray on the polyacrylamide gel on a TLC plate, the DNA fragments are permitted to flow out electrically from the gels cut out by a razor, etc. The effluent containing the DNA fragments is concentrated and then purified by gel filtration column with ®Sephadex G-25 (Pharmacia Inc.), etc.

    iv) The single-strand thus purified is mixed with the other complementary single-strand in a solution, heated at $70°C$ for 5 minutes and then annealed by cooling slowly to room temperature to form a double-stranded DNA. This is used as such for recombination.

(3) Introduction of DNA fragment synthesized:

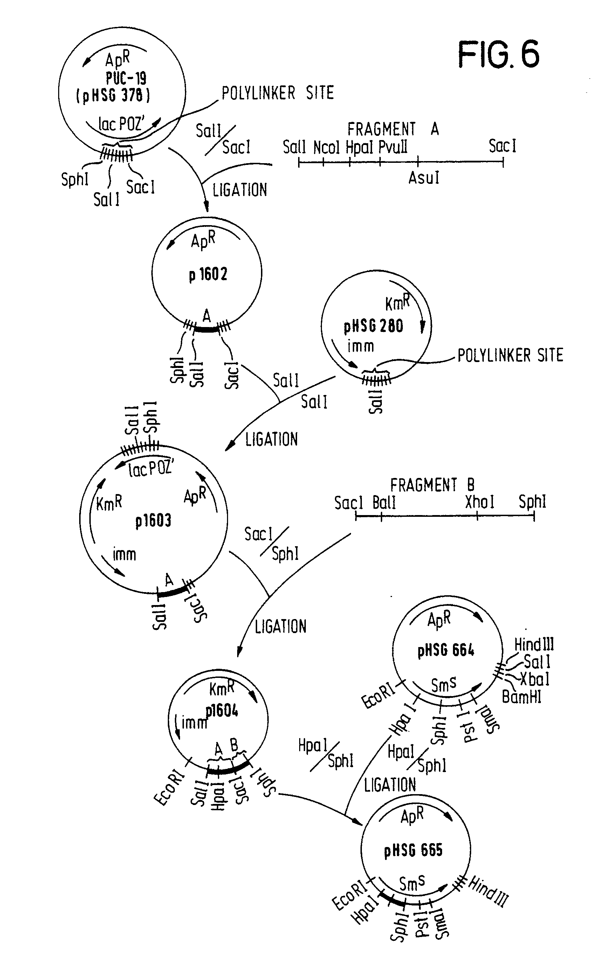

The DNA fragment synthesized is incorporated by utilizing the five recognition sites of HpaI, PvuII, SphI, PstI and SmaI existing in the streptomycin sensitive gene in the pHSG664, the polylinker site comprising BamHI, SalI, XbaI and HindIII existing downstream of said gene or the EcoRI site existing upstream of said gene. Details about introduction of DNA fragments are described in Figs. 6 ~ 9 and Examples 1 ~ 6.

In Example 1, the No. 1 group of recognition sites are introduced between the HpaI and SphI sites in the streptomycin sensitive gene of the pHSG664. In Example 2, the No. 4 group of recognition sites are introduced between the SmaI site in the streptomycin sensitive gene and the HindIII site in the polylinker. In Example 3, the No. 3 group of recognition sites are introduced between the PstI and SmaI sites in the streptomycin sensitive gene. In Example 6, the No. 2 group of recognition sites are introduced between the SphI and PstI sites in the streptomycin sensitive gene. When introducing additional recognition sites other than the No. 1, No. 2, No. 3 and No. 4 groups of recognition sites, the additional sites are incorporated by use of the No. 1, No. 2, No. 3 and No. 4 groups of recognition sites already introduced in addition to the sites as described above.

(4) Determination of base sequence of the synthesized improved type streptomycin sensitive gene:

The synthesized streptomycin sensitive gene is subcloned into the polylinker portion existing locally at the lacPOZ' portion of M13mp18FIDNA or M13mp19RFIDNA (commercially available from Pharmacia Inc.). The recombinant plasmid obtained is read by means of a DNA sequence kit (produced by Takara Shuzo Co., Ltd.) from a synthetic primer by the Klenow Enzyme and, after electrophoresis with 6% polyacrylamide gel, the base sequence is determined from the pattern of autoradiography.

(5) Assay of biological activity of the improved type streptomycin sensitive plasmid vector:

The improved type streptomycin sensitive plasmid vector, including the plasmid vector which is the intermediate during the preparation steps thereof, is checked for its resistance or sensitivity to streptomycin as follows. In an LS medium [a medium containing 10% Bacto tryptone (produced by Difco Laboratories), 5% Bacto yeast extract (produced by Difco Laboratories), 2.5% sodium chloride and 1% glucose, having pH adjusted to 7.2 ~ 7.4 with 1M sodium hydroxide solution] containing 2% agarose (produced by Difco Laboratories, U.S.A.) ampicillin and streptomycin (both produced by Meiji Seika Kaisha, Ltd.) each to a final concentration of 100 μg/ml and solidified on a plastic laboratory dish. Also, for control purpose, an LS medium containing only ampicillin is prepared. On these solid media, the transformed Escherichia coli having said plasmid vector is seeded, cultured at 37° C overnight, and presence of growth of the microorganism on each medium is judged by observation with eyes.

The present invention is described below by referring to Reference example and Examples, which are not limitative of the present invention.

Reference example Preparation of plasmid vector pHSG664 (see Fig. 5)

i) Isolation of Sm$^s$ gene and addition of a polylinker site and EcoRI site thereto:

A plasmid vector pNO1523 commercially available from Pharmacia Inc. in Sweden was digested with SstII and treated with T$_4$DNA polymerase to make SstII a blunt end. Subsequently, this was digested with BamHI and the Sm$^s$ gene was excised from pNO1523.

On the other hand, a plasmid vector pHSG591 (G. Brady et al., Gene, 27 (1984) 223 ~ 232) was digested with PstI and BglII, treated with T$_4$DNA polymerase and then ligated with DNA ligase to have the sites of PstI and BglII contained in the polylinker portion of the pHSG591 disappear. The plasmid vector thus obtained was digested with BamHI and SmaI and linked to the DNA coding for the Sm$^s$ gene previously obtained to obtain pHSG660. The pHSG660 is described by one of the present co-inventors and G. Brady et al. in the above Gene, 27 (1984), 223 ~ 232 and is also available to everybody from Hoechst Japan Ltd., Development & Research Laboratories, or Deutsches Krebsforschungzentrum, Institut für Zell-und Tumorbiologie, Heidelberg. As different from pNO1523, in pHSG660, the sensitive gene (Sm$^s$) has its both ends sandwiched between (a) EcoRI site and (b) a polylinker site comprising BamHI, SalI, XbaI and HindIII sites so that the following recombination can be easily done. The pHSG660 was decomposed with EcoRI and HindIII to excise a fragment containing the Sm$^s$ gene. The fragment was fractionated according to agarose gel electrophoresis and purified through a DE-52 column (Whatman Ltd., U.S.A.) and according to the ethanol precipitation, etc., whereby the Sm$^s$ gene having a polylinker site comprising BamHI, SalI, XbaI and HindIII and EcoRI site added was isolated.

ii) Isolation of ampicillin resistant gene (Ap$^R$) and a pUC replication origin:

The pUC9 (Gene, 19 (1982) 259 ~ 268) commercially available from Pharmacia Inc. in Sweden was digested partially with HaeII then with HindIII, and subsequently the filling in reaction was carried out with T$_4$DNA polymerase to effect linking, thereby obtaining pHSG1311. The cyclic plasmid has the N-end region of the lacZ' gene contained in pUC9 removed therefrom. This was once grown in the host of Escherichia coli, followed by separation and purification. Thus purified cyclic plasmid was subjected to partial restrictive digestion with AhaII, then digested with EcoRI, treated with T₄DNA polymerase to make the ends formed blunt ends, which were then linked together to obtain a cyclic plasmid pHSG367. The plasmid was grown in the host of Escherichia coli, and then separated and purified. The plasmid pHSG367 has all of the region coding for the lacZ' gene except for the polylinker site between the HindIII and EcoRI removed from the original pUC9, and has the ampicillin resistant gene of pUC9 and the pUC replication origin remained therein.

iii) Preparation of a plasmid vector pHSG664 containing Sm$^s$, Ap$^R$ and a replication origin of pUC:

The cyclic plasmid obtained in the step of ii) was decomposed with EcoRI and HindIII, mixed with the DNA fragment containing the streptomycin sensitive gene obtained by decomposition with EcoRI and HindIII in the step of i), and ring closure was effected according to the binding reaction to obtain a new plasmid vector pHSG664. In order to confirm that the various reactions up to this stage have correctly progressed, the base sequence of the plasmid DNA molecule obtained was determined by means of a DNA sequence determining kit (produced by Takara Shuzo Co., Ltd.), etc., or alternatively mapping by decomposition with a plurality of restriction enzymes was performed. As a result, it was confirmed that the desired plasmid having the recognition sites by the restriction enzymes as shown in Fig. 5 was obtained.

Example 1 Introduction of No. 1 group of recognition sites (preparation of pHSG665, see Fig. 6)

Fragments A and B shown in Fig. 3 were synthesized. The fragment A has the sites of SalI, NcoI, HpaI and PvuII added as the sites for linking other than the sites of AsuII and SacI to be introduced. The HpaI and PvuII corresponding to those sites contained in the Sm$^s$ gene of pNO1253, and they are added for making cloning easier. The fragment B has the sites of SacI and SphI as the sites for linking in addition to the recognition sites of BalI and XhoI to be introduced. The fragment A was subjected to subcloning between SalI and SacI of pUC-19 (pHSG378) commercially available from Pharmacia Inc. to obtain a plasmid vector of p1602. The p1602 and the pHSG280 having the kanamycin resistant gene (Km$^R$) (the kanamycin resistant gene is derived from transposon Tn903 [Oka, A. et al.: J. Mol. Biol. 147, 217 ~ 226 (1981)], the polylinker portion is derived from the pUC-8 commercially available from Pharmacia Inc. with XbaI and BglII being further newly added between its HindIII and PstI from synthetic nucleotides) were each digested with SalI and linked together to obtain a plasmid of p1603. The p1603 was cleaved with SacI and SphI and the fragment B was incorporated at that portion whereby p1604 containing a DNA fragment having a group of recognition sites of AsuII, SacI, BalI and XhoI (No. 1 group of recognition sites) and PvuII, HpaI, NcoI and SalI sites was obtained.

The above DNA contained in the p1604 was cleaved with HpaI and SphI and replaced with the portion HpaI-SphI of the pHSG664 previously obtained, whereby a streptomycin sensitive plasmid vector pHSG665 having the No. 1 group of recognition sites was obtained.

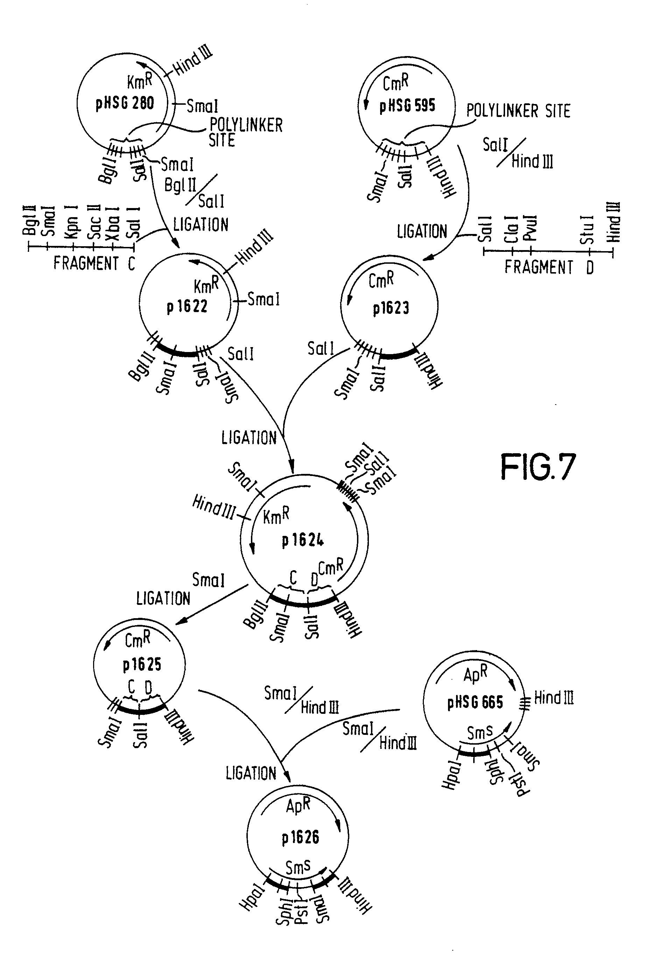

Example 2 Introduction of No. 4 group of recognition sites (preparation of p1626, see Fig. 7)

Fragments C and D shown in Fig. 3 were synthesized. The fragment C has the site of BglII as the site for linking added. The fragment D has HindIII added as the site for linking. In the fragment C, the BglII-SalI portion of the kanamycin resistant pHSG280 was replaced to obtain a plasmid vector p1622. The SalI-HindIII portion of the chloramphenicol-resistant pHSG595 (the chloramphenicol-resistant gene is derived from transposon Tn9 [Alton, N.K., et al.: Nature, 282, 864 ~ 869 (1979)], the polylinker portion and other vector portion are derived from pUC-12 commercially available from Pharmacia Inc.] was replaced to obtain a plasmid vector p1623. Subsequently, both of p1622 and p1623 were cleaved and linked together to obtain a plasmid vector p1624. This was cleaved with SmaI, followed by linking, to obtain a p1625 in which the No. 4 group of recognition sites was introduced on the chloramphenicol-resistant plasmid vector. By cleaving the DNA corresponding to the No. 4 group of recognition sites contained in the p1625 and replacing the cleaved portion with the SmaI-HindIII portion of the pHSG665 previously obtained, a streptomycin resistant plasmid vector p1626 having the No. 1 and No. 4 groups of recognition sites was obtained.

It is self-explanatory that the Sm$^s$ plasmid vector having only the No. 4 series of recognition series introduced therein can be obtained by replacing the DNA corresponding to the No. 4 group of recognition sites with the SmaI-HindIII portion of the pHSG664.

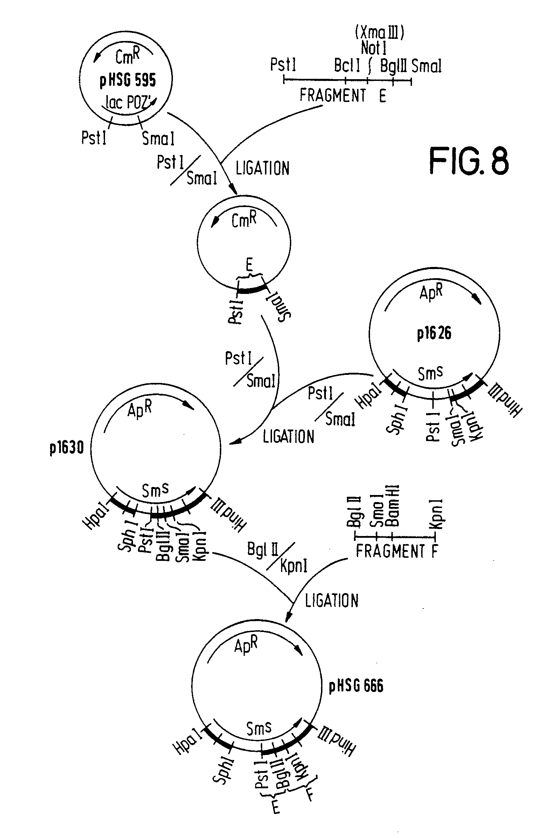

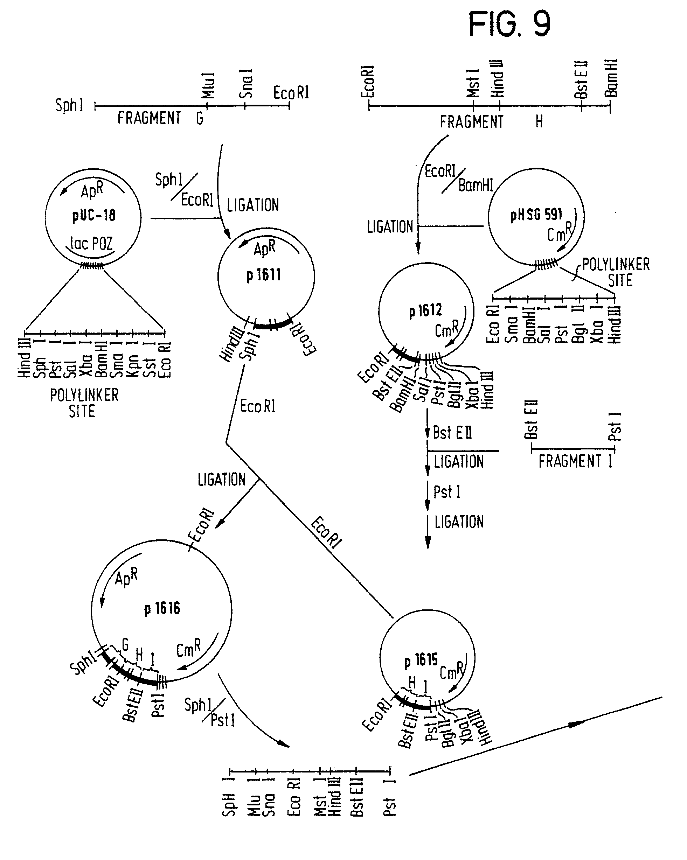

Example 3 Introduction of No. 3 group of recognition sites (preparation of p1630, see Fig. 8)

DNA fragment E corresponding to the No. 3 group of recognition sites shown in Fig. 3 was synthesized. The fragment E has the sites of PstI and SmaI as the site for recombination. The fragment E was once subjected to subcloning with the chloramphenicol-resistant pHSG595, and the same fragment was excised and replaced with the Pst-SmaI portion of the p1626 previously obtained to obtain a Sm$^s$ plasmid vector p1630 having the No. 1, No. 3 and No. 4 groups of recognition sites introduced therein.

It is self-explanatory that a plasmid vector having only the No. 3 group of recognition sites introduced therein can be obtained by introduction of the fragment E into the pHSG664, and also that a plasmid vector having the No. 1 and No. 3 groups of recognition sites introduced therein can be obtained by introduction of the fragment E into the pHSG665.

**Example 4** Introduction of BamHI site into the No. 4 groups of recognition sites (preparation of pHSG666, see Fig. 8)

DNA fragment F shown in Fig. 3 was synthesized. The BglII-KpnI portion of the p1630 obtained in Example 3 was excised by digestion with BglII and KpnI and the synthetic DNA fragment F was ligated with T$_4$DNA ligase thereat to obtain pHSG666 having the No. 1 and 3 groups of recognition sites and the No. 4 group of recognition sites added with BamHI site.

The HB101 strain of Escherichia coli K12 having this plasmid vector pHSG666 incorporated therein is deposited at Fermentation Research Institute, Agency of Industrial Science and Technology under FERM BP-1183.

The pHSG666 has been evidenced to have the streptomycin sensitive gene according to the assaying method shown in the above step (5) and also evidenced to have the base sequence of the programmed improved type streptomycin sensitive gene according to the base sequence determination method in the above step (4). The base sequence and the amino acid sequence of the streptomycin sensitive gene are shown together in Fig. 1. Also, all the cloning sites of the pHSG666 are shown in Fig. 11.

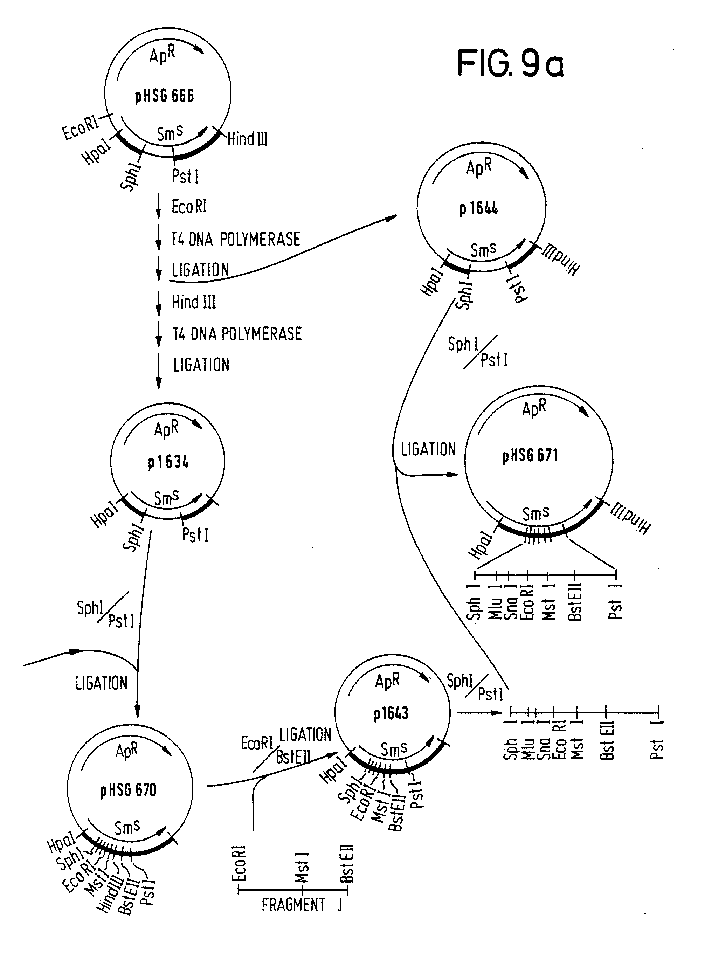

**Example 5** Introduction of the No. 2 group of recognition sites added with HindIII (preparation of pHSG670, see Fig. 9/9a)

DNA fragments G, H and I shown in Fig. 3 were synthesized. The pUC-18 commercially available from Pharmacia Inc. was cleaved with SphI and EcoRI, and the synthetic DNA fragment G

incorporated at the cleaved portion to obtain p1611.

On the other hand, the pHSG591 (G. Brady et al., Gene 27 (1982) 223 ~ 232) was cleaved with EcoRI and BamHI and the synthetic DNA fragment H was incorporated at the cleaved portion to obtain pHSG1612. The pHSG1612 was cleaved with BstEII and the synthetic DNA fragment I was bound thereto. The DNA chain obtained has two PstI sites and, for the purpose of making the PstI unique by removing the portion existing between both the sites, the DNA chain obtained was digested with PstI and combined again to obtain a plasmid vector p1615. Both of the p1611 and the p1615 were cleaved with EcoRI and bound together to obtain P1616. The p1616 has the No. 2 group of recognition sites and the HindIII site between EcoRI and BstEII thereof.

On the other hand, since the pHSG666 obtained in Example 4 contains the EcoRI and HindIII contained in the No. 2 group of recognition sites on the outside of the Sm$^s$ gene, it is desirable to delete the outside EcoRI and HindIII sites prior to recombination. Accordingly, the pHSG666 was digested with EcoRI, filling in with T$_4$DNA polymerase and then ligated with DNA ligase to delete the EcoRI site. Subsequently, also the HindIII site was deleted by digestion with HindIII, filling in with T$_4$DNA polymerase and ligated with DNA ligase to obtain p1634.

By digesting the p1616 previously obtained with SphI and PstI, a fragment for replacement was excised. The p1634 was digested with SphI and PstI and the previous fragment was bound thereto to obtain a pHSG670 having the No. 2 group of recognition sites and additively the HindIII site between EcoRI and BstEII thereof. It is self-explanatory that this fragment can be utilized for replacement of the "SphI-PstI" portion of the pHSG664, the pHSG665, the p1626 and the p1630.

**Example 6** Introduction of the No. 2 group of recognition sites (preparation of pHSG671, see Fig. 9/9a)

DNA fragment J shown in Fig. 3 was synthesized. This is a fragment for deleting the HindIII site from the pHSG670. The pHSG670 was digested with EcoRI and BstEII and the synthetic DNA fragment J was replaced at the digested portion to obtain p1643 having the No. 2 group of recognition sites. By digestion of p1643 with SphI and PstI, a fragment having the No. 2 group of recognition sites was excised.

On the other hand, from the pHSG666, p1644 without the EcoRI outside of the Sm$^s$ gene was obtained according to the method as described in Example 5. p1644 was digested with SphI and PstI, and the fragment excised as described above was then linked to the "SphI-PstI" site to obtain pHSG671. The HB101 strain of Escherichia coli K12 having the plasmid vector pHSG671 incorporated therein is deposited at Fermentation Research Institute, Agency of Industrial Science and Technology under FERM BP-1184. It is self-explanatory that the fragment having the No. 2 group of recognition sites can be utilized for replacement of the "SphI-PstI" portion of the pHSG664, the pHSG665, the pHSG1626 and the p1630.

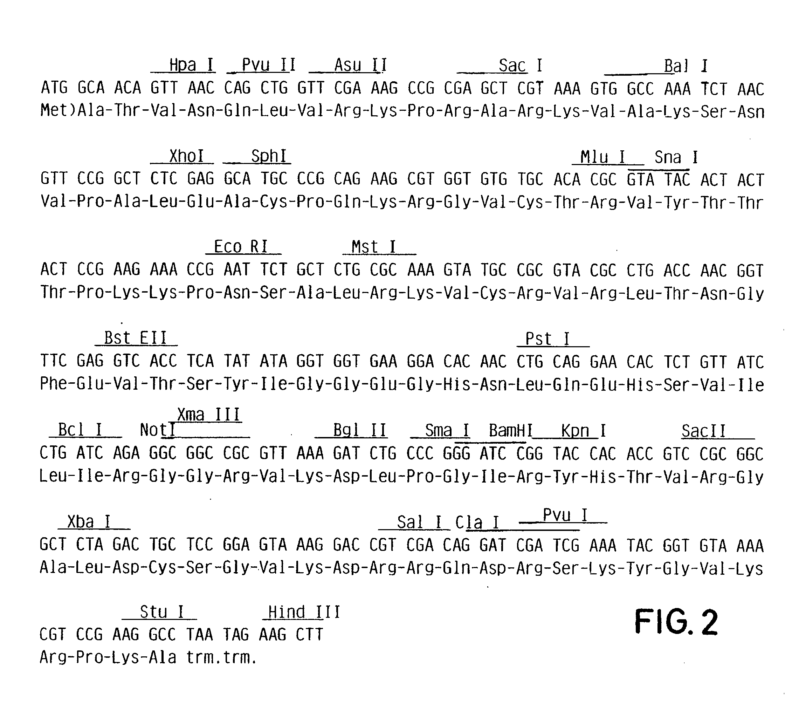

The base sequence of the streptomycin sensitive gene of the pHSG671 has been determined according to the above step 4 and is shown together with the amino acid sequence in Fig. 2.

Example 7 Use of the improved type streptomycin sensitive plasmid vector

Between PstI and BglII of the pHSG666 of the improved type streptomycin sensitive plasmid vector prepared by the present invention, a foreign "PstI-BglII" DNA fragment was inserted by recombination, Escherichia coli transformed with this recombinant plasmid and grown in an LS selective medium containing each 100 μg/ml of ampicillin and streptomycin to obtain streptomycin resistant strains in number of 4 x 10⁻⁴ (transformants/μg of plasmid DNA). From the colonies of these resistant strains, 14 colonies were randomly selected and the plasmid DNA was extracted from each of them and digested with PstI and BglII. As a result , it was evidenced that the foreign DNA fragment was incorporated correctly between PstI and BglII of the pHSG666 in all of 14 colonies.

Brief Description of the Drawings
Fig. 1 shows the base sequence of pHSG666;
Fig. 2 shows the base sequence of pHSG671;
Fig. 3 shows a list of the synthetic DNA fragments;

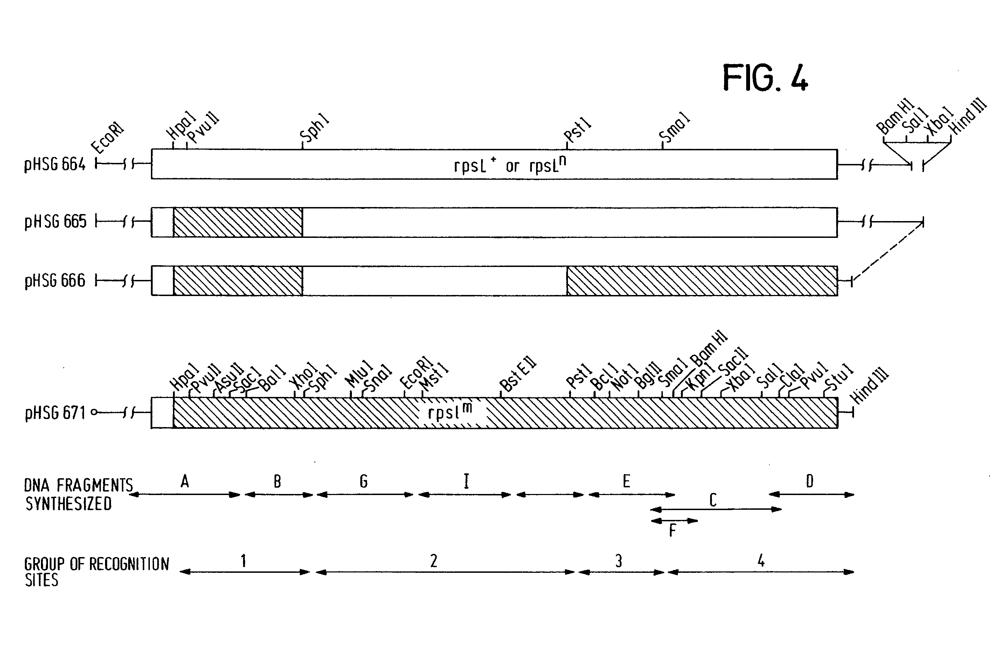

FIG. 4 illustrates correspondence synthetic DNA fragments to pHSG671, rpsLⁿ and rpsLᵐ represent natural and modified streptomycin sensitive gene respectively;
Fig. 5 illustrates preparation of pHSG664;
Fig. 6 illustrates preparation of pHSG665;
Fig. 7 illustrates preparation of pHSG1626;
Fig. 8 illustrates preparation of pHSG1630 and pHSG666;
Fig. 9/9a illustrate preparation of pHSG670 and pHSG671;
Fig. 10 shows comparison between the bases of pNO1523 and pHSG671;
Fig. 11 shows restriction enzyme map of pHSG666; and Fig. 12 shows restriction enzyme map of pHSG671.

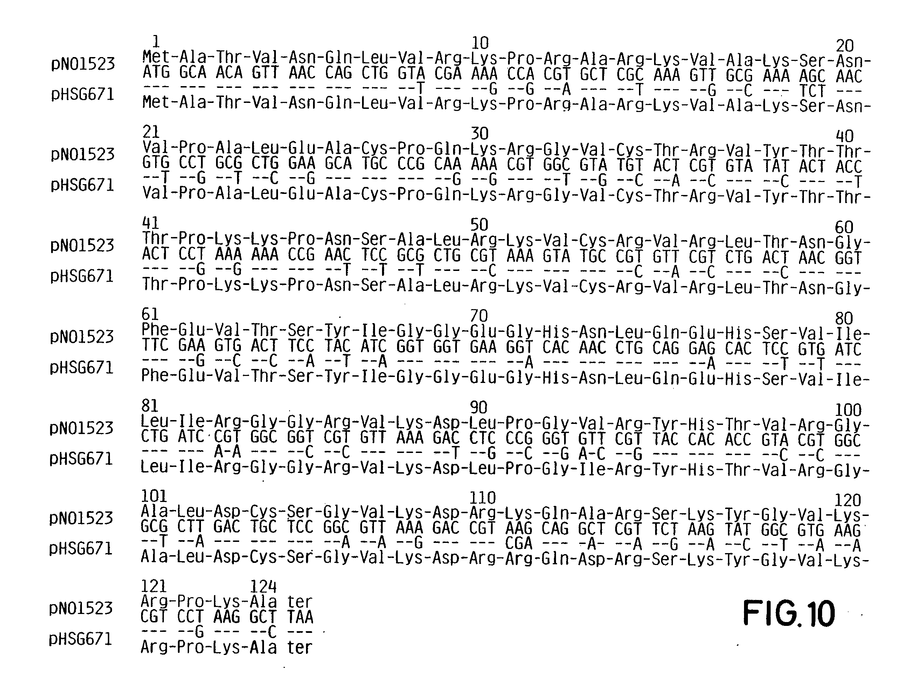

In Figs. 5 ~ 10, the portions written in bold lines indicate the portions replaced with synthetic DNA fragments.

Claims

1. A streptomycin sensitive gene (rps L⁺) shown in Figure 1, where recognition sites of restriction enzymes have been introduced by partially changing the base sequence of the streptomycin sensitive gene without changing the characteristics of the streptomycin sensitivity, having at least one group of recognition sites selected from
   a group of restriction enzyme recognition sites consisting of AsuII, SacI, BalI and XhoI (hereinafter called No. 1 group of recognition sites);
   a group of restriction enzyme recognition sites consisting of MluI, SnaI, EcoRI, MstI and BstEII (hereinafter called No. 2 group of recognition sites);
   a group of restriction enzyme recognition sites consisting of BclI, NotI and BglII (hereinafter called No. 3 group of recognition sites); and
   a group of restriction enzyme recognition sites consisting of KpnI, SacII, XbaI, SalI, ClaI, PvuI and StuI (hereinafter called No. 4 group of recognition sites).

2. A streptomycin sensitive gene according to claim 1,
   having additionally a recognition site of restriction enzyme BamHI.

3. A streptomycin sensitive gene according to claim 2,
   having No. 1, 3 and 4 groups of recognition sites and the BamHI site.

4. A streptomycin sensitive gene according to claim 2,
   having all of the No. 1, 2, 3 and 4 groups of recognition sites and the BamHI site.

5. A plasmid vector containing a streptomycin sensitive gene (rps L⁺) shown in Figure 1, where recognition sites of restriction enzymes have been introduced by partially changing the base sequence of the streptomycin sensitive gene without changing the characteristics of the streptomycin sensitivity, having at least one group of recognition sites selected from

    a group of restriction enzyme recognition sites consisting of AsuII, SacI, BalI and XhoI (hereinafter called No. 1 group of recognition sites);

    a group of restriction enzyme recognition sites consisting of MluI, SnaI, EcoRI, MstI and BstEII (hereinafter called No. 2 group of recognition sites);

    a group of restriction enzyme recognition sites consisting of BclI, NotI and BGlII (hereinafter called No. 3 group of recognition sites); and

    a group of restriction enzyme recognition sites consisting of KpnI, SacII, XbaI, SalI, ClaI, PvuI and StuI (hereinafter called No. 4 group of recognition sites).

6. A plasmid vector according to claim 5, containing a streptomycin sensitive gene having additionally a recognition site of restriction enzyme BamHI.

7. A plasmid vector according to claim 6, which is designated as pHSG666 (FERM BP-1183).

8. A plasmid vector according to claim 7, which is designated as pHSG671 (FERM BP-1184).

9. A microorganism containing a plasmid vector containing a streptomycin sensitive gene (rps $L^+$) shown in Figure 1, where recognition sites of restriction enzymes have been introduced by partially changing the base sequence of the streptomycin sensitive gene without changing the characteristics of the streptomycin sensitivity, having at least one group of recognition sites selected from

    a group of restriction enzyme recognition sites consisting of AsuII, SacI, BalI and XhoI (hereinafter called No. 1 group of recognition sites);

    a group of restriction enzyme recognition sites consisting of MluI, SnaI, EcoRI, MstI and BstEII (hereinafter called No. 2 group of recognition sites);

    a group of restriction enzyme recognition sites consisting of BclI, NotI and GblII (hereinafter called No. 3 group of recognition sites); and

    a group of restriction enzyme recognition sites consisting of KpnI, SacII, XbaI, SalI, ClaI, PvuI and StuI (hereinafter called No. 4 group of recognition sites).

10. A microorganism according to claim 9, containing a plasmid vector which is designated as plasmid pHSG666 (FERM BP-1183).

11. A microorganism according to claim 9, containing a plasmid vector which is designated as plasmid pHSG671 (FERM BP-1184).

Claims for the following Contracting States : AT, ES

1. A process for preparing a streptomycin sensitive gene (rps $L^+$) shown in Figure 1, where recognition sites of restriction enzymes have been introduced by partially changing the base sequence of the streptomycin sensitive gene without changing the characteristics of the streptomycin sensitivity, which comprises inserting into the gene at least one group of recognition sites selected from:

    a group of restriction enzyme recognition sites consisting of AsuII, SacI, BalI and XhoI (hereinafter called No. 1 group of recognition sites);

    a group of restriction enzyme recognition sites consisting of MluI, SnaI EcoRI, MstI and BstEII (hereinafter called No. 2 group of recognition sites);

    a group of restriction enzyme recognition sites consisting of BclI, NotI and BglII (hereinafter called No. 3 group of recognition sites); and

    a group of restriction enzyme recognition sites consisting of KpnI, SacII, XbaI, SalI, ClaI, PvuI and StuI (hereinafter called No. 4 group of recognition sites).

2. A process according to claim 1, which comprises inserting into the gene additionally a recognition site of the restriction enzyme BamHI.

3. A process according to claim 1, which comprises inserting into the gene No. 1, 3 and 4 groups of recognition sites and the BamHI site.

4. A process according to claim 2, which comprises inserting into the gene all of the No. 1, 2, 3 and 4 groups of recognition sites and the BamHI site.

5. A process for modifying a plasmid vector containing a streptomycin sensitive gene (rps $L^+$) shown in Figure 1, where recognition sites of restriction enzymes have been introduced by partially changing the base sequence of the streptomycin sensitive gene without changing the characteristics of the streptomycin sensitivity, which comprises inserting into the gene at least one group of recognition sites selected from:

    a group of restriction enzyme recognition sites consisting of AsuII, SacI, BalI and XhoI (hereinafter called No. 1 group of recognition sites);

a group of restriction enzyme recognition sites consisting of MluI, SnaI, EcoRI, MstI and BstEII (hereinafter called No. 2 group of recognition sites);

a group of restriction enzyme recognition sites consisting of BclI, NotI and BglII (hereinafter called No. 3 group of recognition sites); and

a group of restriction enzyme recognition sites consisting of KpnI, SacII, XbaI, SalI, ClaI, PvuI and StuI (hereinafter called No. 4 group of recognition sites).

6. A process according to claim 5, which comprises inserting into the streptomycin sensitive gene additionally a recognition site of the restriction enzyme BamHI.

7. A process according to claim 6, which results in the vector designated as pHSG666 (FERM BP-1183).

8. A process according to claim 7, which results in the vector designated as pHSG671 (FERM BP-1184).

9. A process for transforming a microorganism which comprises incorporating into the microorganism a plasmid vector containing a streptomycin sensitive gene (rps L$^+$) shown in Figure 1, where recognition sites of restriction enzymes have been introduced by partially changing the base sequence of the streptomycin sensitive gene without changing the characteristics of the streptomycin sensitivity, which comprises inserting into the gene at least one group of recognition sites selected from:

a group of restriction enzyme recognition sites consisting of AsuII, SacI, BalI and XhoI (hereinafter called No. 1 group of recognition sites);

a group of restriction enzyme recognition sites consisting of MluI, SnaI, EcoRI, MstI and BstEII (hereinafter called No. 2 group of recognition sites);

a group of restriction enzyme recognition sites consisting of BclI, NotI and BglII (hereinafter called No. 3 group of recognition sites); and

a group of restriction enzyme recognition sites consisting of KpnI, SacII, XbaI, SalI, ClaI, PvuI and StuI (hereinafter called No. 4 group of recognition sites).

10. A process according to claim 9, which comprises incorporating into the microorganism a plasmid vector which is designated as plasmid pHSG666 (FERM BP-1183).

11. A process according to claim 9, which comprises incorporating into the microorganism a plasmid vector which is designated as plasmid pHSG671 (FERM BP-1184).

**Revendications**

1. Gène sensible à la streptomycine (rps L$^+$) représenté sur la figure 1, dans lequel des sites de reconnaissance d'enzymes de restriction ont été introduits par modification partielle de la séquence de bases du gène sensible à la streptomycine sans altération des caractéristiques de la sensibilité à la streptomycine, comportant au moins un groupe de sites de reconnaissance choisi parmi

un groupe de sites de reconnaissance d'enzymes de restriction constitué de AsuII, SacI, BalI et XhoI (dénommé ci-après "groupe n° 1 de sites de reconnaissance");

un groupe de sites de reconnaissance d'enzymes de restriction constitué de MluI, SnaI, EcoRI, MstI et BstEII (dénommé ci-après "groupe n° 2 de sites de reconnaissance");

un groupe de sites de reconnaissance d'enzymes de restriction constitué de BclI, NotI et BglII (dénommé ci-après "groupe n° 3 de sites de reconnaissance"); et

un groupe de sites de reconnaissance d'enzymes de restriction constitué de KpnI, SacII, XbaI, SalI, ClaI, PvuI et StuI (dénommé ci-après "groupe n° 4 de sites de reconnaissance").

2. Gène sensible à la streptomycine selon la revendication 1, comportant en outre un site de reconnaissance d'enzyme de restriction BamHI.

3. Gène sensible à la streptomycine selon la revendication 2, comportant les groupes n° 1, 3 et 4 de sites de reconnaissance et le site BamHI.

4. Gène sensible à la streptomycine selon la revendication 2, comportant tous les groupes n° 1, 2, 3 et 4 de sites de reconnaissance et le site BamHI.

5. Vecteur plasmidique contenant un gène sensible à la streptomycine (rps L$^+$) représenté sur la figure 1, dans lequel des sites de reconnaissance d'enzymes de restriction ont été introduits par modification partielle de la séquence de bases du gène sensible à la streptomycine sans altération des caractéristiques de la sensibilité à la streptomycine, comportant au moins un groupe de sites de reconnaissance choisi parmi

un groupe de sites de reconnaissance d'enzymes de restriction constitué de AsuII, SacI, BaII et XhoI (dénommé ci-après "groupe n° 1 de sites de reconnaissance");

un groupe de sites de reconnaissance d'enzymes de restriction constitué de MluI, SnaI, EcoRI, MstI et BstEII (dénommé ci-après "groupe n° 2 de sites de reconnaissance");

un groupe de sites de reconnaissance d'enzymes de restriction constitué de BcII, NotI et BgIII (dénommé ci-après "groupe n° 3 de sites de reconnaissance"); et

un groupe de sites de reconnaissance d'enzymes de restriction constitué de KpnI, SacII, XbaI, SaII, ClaI, PvuI et StuI (dénommé ci-après "groupe n° 4 de sites de reconnaissance").

6. Vecteur plasmidique selon la revendication 5, contenant un gène sensible à la streptomycine comportant en outre un site de reconnaissance d'enzyme de restriction BamHI.

7. Vecteur plasmidique selon la revendication 6, qui est désigné par pHSG666 (FERM BP-1183).

8. Vecteur plasmidique selon la revendication 6, qui est désigné par pHSG671 (FERM BP-1184).

9. Micro-organisme contenant un vecteur plasmidique contenant un gène sensible à la streptomycine (rps $L^+$) représenté sur la figure 1, dans lequel des sites de reconnaissance d'enzymes de restriction ont été introduits par modification partielle de la séquence de bases du gène sensible à la streptomycine sans altération des caractéristiques de la sensibilité à la streptomycine, comportant au moins un groupe de sites de reconnaissance choisi parmi

un groupe de sites de reconnaissance d'enzymes de restriction constitué de AsuII, SacI, BaII et XhoI (dénommé ci-après "groupe n° 1 de sites de reconnaissance");

un groupe de sites de reconnaissance d'enzymes de restriction constitué de MluI, SnaI, EcoRI, MstI et BstEII (dénommé ci-après "groupe n° 2 de sites de reconnaissance");

un groupe de sites de reconnaissance d'enzymes de restriction constitué de BcII, NotI et BgIII (dénommé ci-après "groupe n° 3 de sites de reconnaissance"); et

un groupe de sites de reconnaissance d'enzymes de restriction constitué de KpnI, SacII, XbaI, SaII, ClaI, PvuI et StuI (dénommé ci-après "groupe n° 4 de sites de reconnaissance").

10. Micro-organisme selon la revendication 9, contenant un vecteur plasmidique qui est dénommé "plasmide pHSG666" (FERM BP-1183).

11. Micro-organisme selon la revendication 9, contenant un vecteur plasmidique qui est dénommé "plasmide pHSG671" (FERM BP-1184).

Revendications pour les Etats contractants suivants : AT, ES

1. Procédé pour la production d'un gène sensible à la streptomycine (rps $L^+$) représenté sur la figure 1, dans lequel des sites de reconnaissance d'enzymes de restriction ont été introduits par modification partielle de la séquence de bases du gène sensible à la streptomycine sans altération des caractéristiques de la sensibilité à la streptomycine, lequel comprend l'insertion dans le gène d'au moins un groupe de sites de reconnaissance choisi parmi:

un groupe de sites de reconnaissance d'enzymes de restriction constitué de AsuII, SacI, BaII et XhoI (dénommé ci-après "groupe n° 1 de sites de reconnaissance");

un groupe de sites de reconnaissance d'enzymes de restriction constitué de MluI, SnaI, EcoRI, MstI et BstEII (dénommé ci-après "groupe n° 2 de sites de reconnaissance");

un groupe de sites de reconnaissance d'enzymes de restriction constitué de BcII, NotI et BgIII (dénommé ci-après "groupe n° 3 de sites de reconnaissance"); et

un groupe de sites de reconnaissance d'enzymes de restriction constitué de KpnI, SacII, XbaI, SaII, ClaI, PvuI et StuI (dénommé ci-après "groupe n° 4 de sites de reconnaissance").

2. Procédé selon la revendication 1, qui comprend l'insertion dans le gène, en outre, d'un site de reconnaissance d'enzyme de restriction BamHI.

3. Procédé selon la revendication 1, qui comprend l'insertion dans le gène des groupes n° 1, 3 et 4 de sites de reconnaissance et du site BamHI.

4. Procédé selon la revendication 1, qui comprend l'insertion dans le gène de tous les groupes n° 1, 2, 3 et 4 de sites de reconnaissance et du site BamHI.

5. Procédé pour la modification d'un vecteur plasmidique contenant un gène sensible à la streptomycine (rps L$^+$) représenté sur la figure 1, dans lequel des sites de reconnaissance d'enzymes de restriction ont été introduits par modification partielle de la séquence de bases du gène sensible à la streptomycine sans altération des caractéristiques de la sensibilité à la streptomycine, qui comprend l'insertion dans le gène d'au moins un groupe de sites de reconnaissance choisi parmi:

un groupe de sites de reconnaissance d'enzymes de restriction constitué de AsuII, SacI, BalI et XhoI (dénommé ci-après "groupe n° 1 de sites de reconnaissance");

un groupe de sites de reconnaissance d'enzymes de restriction constitué de MluI, SnaI, EcoRI, MstI et BstEII (dénommé ci-après "groupe n° 2 de sites de reconnaissance");

un groupe de sites de reconnaissance d'enzymes de restriction constitué de BclI, NotI et BglII (dénommé ci-après "groupe n° 3 de sites de reconnaissance"); et

un groupe de sites de reconnaissance d'enzymes de restriction constitué de KpnI, SacII, XbaI, SalI, ClaI, PvuI et StuI (dénommé ci-après "groupe n° 4 de sites de reconnaissance").

6. Procédé selon la revendication 5, qui comprend l'insertion dans le gène sensible à la streptomycine, en outre, d'un site de reconnaissance de l'enzyme de restriction BamHI.

7. Procédé selon la revendication 6, ayant pour résultat le vecteur désigné par pHSG666 (FERM BP-1183).

8. Procédé selon la revendication 6, ayant pour résultat le vecteur désigné par pHSG671 (FERM BP-1184).

9. Procédé pour la transformation d'un micro-organisme, qui comprend l'incorporation dans le micro-organisme d'un vecteur plasmidique contenant un gène sensible à la streptomycine (rps L$^+$) représenté sur la figure 1, dans lequel des sites de reconnaissance d'enzymes de restriction ont été introduits par modification partielle de la séquence de bases du gène sensible à la streptomycine sans altération des caractéristiques de la sensibilité à la streptomycine, qui comprend l'insertion dans le gène d'au moins un groupe de sites de reconnaissance choisi parmi:

un groupe de sites de reconnaissance d'enzymes de restriction constitué de AsuII, SacI, BalI et XhoI (dénommé ci-après "groupe n° 1 de sites de reconnaissance");

un groupe de sites de reconnaissance d'enzymes de restriction constitué de MluI, SnaI, EcoRI, MstI et BstEII (dénommé ci-après "groupe n° 2 de sites de reconnaissance");

un groupe de sites de reconnaissance d'enzymes de restriction constitué de BclI, NotI et BglII (dénommé ci-après "groupe n° 3 de sites de reconnaissance"); et

un groupe de sites de reconnaissance d'enzymes de restriction constitué de KpnI, SacII, XbaI, SalI, ClaI, PvuI et StuI (dénommé ci-après "groupe n° 4 de sites de reconnaissance").

10. Procédé selon la revendication 9, qui comprend l'incorporation dans le micro-organisme d'un vecteur plasmidique qui est dénommé "plasmide pHSG666" (FERM BP-1183).

11. Procédé selon la revendication 9, qui comprend l'incorporation dans le micro-organisme d'un vecteur plasmidique qui est dénommé "plasmide pHSG671" (FERM BP-1184).

## Ansprüche

1. In der Fig. 1 dargestelltes Streptomycin-empfindliches Gen (rps L$^+$), in welches Erkennungsstellen von Restriktionsenzymen durch teilweises Verändern der Basensequenz des Streptomycin-empfindlichen Gens ohne Veränderung der Kennmerkmale der Streptomycin-Empfindlichkeit eingeführt worden sind, und welches wenigstens eine Gruppe von Erkennungsstellen aufweist, welche aus:

einer Gruppe von Restriktionsenzym-Erkennungsstellen, welche aus AsuII-, SacI-, BalI- und XhoI-Erkennungsstellen (nachstehend die Gruppe Nr. 1 von Erkennungsstellen genannt) besteht;

einer Gruppe von Restriktionsenzym-Erkennungsstellen, welche aus MluI-, SnaI-, EcoRI-, MstI- und BstEII-Erkennungsstellen (nachstehend die Gruppe Nr. 2 von Erkennungsstellen genannt) besteht;

einer Gruppe von Restriktionsenzym-Erkennungsstellen, welche aus BclI-, NotI- und BglII-Erkennungsstellen (nachstehend die Gruppe Nr. 3 von Erkennungsstellen genannt) besteht; und

einer Gruppe von Restriktionsenzym-Erken-

nungsstellen, welche aus Kpnl-, Sacll-, Xbal-, Sall-, Clal-, Pvul- und Stul-Erkennungsstellen (nachstehend die Gruppe Nr. 4 von Erkennungsstellen genannt) besteht; ausgewählt ist.

2. Streptomycin-empfindliches Gen nach Anspruch 1, welches zusätzlich eine Erkennungsstelle des Restriktionsenzyms BamHI aufweist.

3. Streptomycin-empfindliches Gen nach Anspruch 2, welches die Gruppen Nrn. 1, 3 und 4 von Erkennungsstellen und die BamHI-Stelle aufweist.

4. Streptomycin-empfindliches Gen nach Anspruch 2, welches alle Gruppen der Nummern 1, 2, 3 und 4 von Erkennungsstellen und die BamHI-Stelle aufweist.

5. Plasmidvektor, welcher ein in der Fig. 1 dargestelltes, Streptomycin-empfindliches Gen (rps L$^+$) enthält, in welches Erkennungsstellen von Restriktionsenzymen durch teilweises Verändern der Basensequenz des Streptomycin-empfindlichen Gens ohne Veränderung der Kennmerkmale der Streptomycin-Empfindlichkeit eingeführt worden sind, und welches wenigstens eine Gruppe von Erkennungsstellen aufweist, welche aus:
einer Gruppe von Restriktionsenzym-Erkennungsstellen, welche aus Asull-, Sacl-, Ball- und Xhol-Erkennungsstellen (nachstehend die Gruppe Nr. 1 von Erkennungsstellen genannt) besteht;
einer Gruppe von Restriktionsenzym-Erkennungsstellen, welche aus Mlul-, Snal- EcoRl-, Mstl- und BstEll-Erkennungsstellen (nachstehend die Gruppe Nr. 2 von Erkennungsstellen genannt) besteht;
einer Gruppe von Restriktionsenzym-Erkennungsstellen, welche aus Bcll-, Notl- und Bglll-Erkennungsstellen (nachstehend die Gruppe Nr. 3 von Erkennungsstellen genannt) besteht; und
einer Gruppe von Restriktionsenzym-Erkennungsstellen, welche aus Kpnl-, Sacll-, Xbal-, Sall-, Clal-, Pvul- und Stul-Erkennungsstellen (nachstehend die Gruppe Nr. 4 von Erkennungsstellen genannt) besteht; ansgewählt ist.

6. Plasmidvektor nach Anspruch 5, welcher ein Streptomycin-empfindliches Gen enthält, welches zusätzlich eine Erkennungsstelle des Restriktionsenzyms BamHI aufweist.

7. Plasmidvektor nach Anspruch 6, welcher als pHSG666 (FERM BP-1183) bezeichnet wird.

8. Plasmidvektor nach Anspruch 6, welcher als pHSG671 (FERM BP-1184) bezeichnet wird.

9. Mikroorganismus, welcher einen Plasmidvektor enthält, welcher seinerseits ein in der Fig. 1 dargestelltes, Streptomycin-empfindliches Gen (rps L$^+$) enthält, in welches Erkennungsstellen von Restriktionsenzymen durch teilweises Verändern der Basensequenz des Streptomycin-empfindlichen Gens ohne Veränderung der Kennmerkmale der Streptomycin-Empfindlichkeit eingeführt worden sind, und welches wenigstens ein Gruppe von Erkennungsstellen aufweist, welche aus:
einer Gruppe von Restriktionsenzym-Erkennungsstellen, welche aus Asull-, Sacl-, Ball- und Xhol-Erkennungsstellen (nachstehend die Gruppe Nr. 1 von Erkennungsstellen genannt) besteht;
einer Gruppe von Restriktionsenzym-Erkennungsstellen, welche aus Mlul-, Snal- EcoRl-, Mstl- und BstEll-Erkennungsstellen (nachstehend die Gruppe Nr. 2 von Erkennungsstellen genannt) besteht;
einer Gruppe von Restriktionsenzym-Erkennungsstellen, welche aus Bcll-, Notl- und Bglll-Erkennungsstellen (nachstehend die Gruppe Nr. 3 von Erkennungsstellen genannt) besteht; und
einer Gruppe von Restriktionsenzym-Erkennungsstellen, welche aus Kpnl-, Sacll-, Xbal-, Sall-, Clal-, Pvul- und Stul-Erkennungsstellen (nachstehend die Gruppe Nr. 4 von Erkennungsstellen genannt) besteht;
ausgewählt ist.

10. Mikroorganismus nach Anspruch 9, welcher einen als Plasmid pHSG666 (FERM BP-1183) bezeichneten Plasmidvektor enthält.

11. Mikroorganismus nach Anspruch 9, welcher einen als Plasmid pHSG671 (FERM BP-1184) bezeichneten Plasmidvektor enthält.

Patentansprüche für folgende Vertragsstaaten : AT, ES

1. Verfahren zur Herstellung eines in der Fig. 1 dargestellten, Streptomycin-empfindlichen Gens (rps L$^+$), in welches Erkennungsstellen von Restriktionsenzymen durch teilweises Verändern der Basensequenz des Streptomycin-empfindlichen Gens ohne Veränderung der Kennmerkmale der Streptomycin-Empfindlichkeit eingeführt worden sind, und welches Verfahren das Insertieren von wenigstens einer Gruppe von Erkennungsstellen in das Gen umfaßt, welche aus:

einer Gruppe von Restriktionsenzym-Erkennungsstellen, welche aus AsuII-, SacI-, BalI- und XhoI-Erkennungsstellen (nachstehend die Gruppe Nr. 1 von Erkennungsstellen genannt) besteht;

einer Gruppe von Restriktionsenzym-Erkennungsstellen, welche aus MluI-, SnaI- EcoRI-, MstI- und BstEII-Erkennungsstellen (nachstehend die Gruppe Nr. 2 von Erkennungsstellen genannt) besteht;

einer Gruppe von Restriktionsenzym-Erkennungsstellen, welche aus BclI-, NotI- und BglII-Erkennungsstellen (nachstehend die Gruppe Nr. 3 von Erkennungsstellen genannt) besteht; und

einer Gruppe von Restriktionsenzym-Erkennungsstellen, welche aus KpnI-, SacII-, XbaI-, SalI-, ClaI-, PvuI- und StuI-Erkennungsstellen (nachstehend die Gruppe Nr. 4 von Erkennungsstellen genannt) besteht; ansgewählt ist.

2. Verfahren nach Anspruch 1, welches das zusätzliche Insertieren einer Erkennungsstelle des Restriktionsenzyms BamHI in das Gen umfaßt.

3. Verfahren nach Anspruch 1, welches das Insertieren der Gruppen Nrn. 1, 3 und 4 von Erkennungsstellen, und der BamHI-Stelle, in das Gen umfaßt.

4. Verfahren nach Anspruch 2, welches das Insertieren aller Gruppen der Nummern 1, 2, 3 und 4 von Erkennungsstellen, und der BamHI-Stelle, in das Gen umfaßt.

5. Verfahren zum Modifizieren eines Plasmidvektors, welcher ein in der Fig. 1 dargestelltes, Streptomycin-empfindliches Gen (rps L$^+$) enthält, in welches Erkennungsstellen von Restriktionsenzymen durch teilweises Verändern der Basensequenz des Streptomycin-empfindlichen Gens ohne Veränderung der Kennmerkmale der Streptomycin-Empfindlichkeit eingeführt worden sind, und welches Verfahren das Insertieren von wenigstens einer Gruppe von Erkennungsstellen in das Gen umfaßt, welche aus:

einer Gruppe von Restriktionsenzym-Erkennungsstellen, welche aus AsuII-, SacI-, BalI- und XhoI-Erkennungsstellen (nachstehend die Gruppe Nr. 1 von Erkennungsstellen genannt) besteht;

einer Gruppe von Restriktionsenzym-Erkennungsstellen, welche aus MluI-, SnaI- EcoRI-, MstI- und BstEII-Erkennungsstellen (nachstehend die Gruppe Nr. 2 von Erkennungsstellen genannt) besteht;

einer Gruppe von Restriktionsenzym-Erkennungsstellen, welche aus BclI-, NotI- und BglII-Erkennungsstellen (nachstehend die Gruppe Nr. 3 von Erkennungsstellen genannt) besteht; und

einer Gruppe von Restriktionsenzym-Erkennungsstellen, welche aus KpnI-, SacII-, XbaI-, SalI-, ClaI-, PvuI- und StuI-Erkennungsstellen (nachstehend die Gruppe Nr. 4 von Erkennungsstellen genannt) besteht; ausgewählt ist.

6. Verfahren nach Anspruch 5, welches das zusätzliche Insertieren einer Erkennungsstelle des Restriktionsenzyms BamHI in das Streptomycinempfindliche Gen umfaßt.

7. Verfahren nach Anspruch 6, welches zu dem als pHSG666 (FERM BP-1183) bezeichneten Vektor führt.

8. Verfahren nach Anspruch 6, welches zu dem als pHSG671 (FERM BP-1184) bezeichneten Vektor führt.

9. Verfahren zum Transformieren eines Mikroorganismus, welches das Einverleiben eines Plasmidvektors in den Mikroorganismus umfaßt, welcher Plasmidvektor ein in der Fig. 1 dargestelltes, Streptomycin-empfindliches Gen (rps L$^+$) enthält, in welches Erkennungsstellen von Restriktionsenzymen durch teilweises Verändern der Basensequenz des Streptomycin-empfindlichen Gens ohne Veränderung der Kennmerkmale der Streptomycin-Empfindlichkeit eingeführt worden sind, und welches Verfahren das Insertieren von wenigstens einer Gruppe von Erkennungsstellen in das Gen umfaßt, welche aus:

einer Gruppe von Restriktionsenzym-Erkennungsstellen, welche aus AsuII-, SacI-, BalI- und XhoI-Erkennungsstellen (nachstehend die Gruppe Nr. 1 von Erkennungsstellen genannt) besteht;

einer Gruppe von Restriktionsenzym-Erkennungsstellen, welche aus MluI-, SnaI- EcoRI-, MstI- und BstEII-Erkennungsstellen (nachstehend die Gruppe Nr. 2 von Erkennungsstellen genannt) besteht;

einer Gruppe von Restriktionsenzym-Erkennungsstellen, welche aus BclI-, NotI- und BglII-Erkennungsstellen (nachstehend die Gruppe Nr. 3 von Erkennungsstellen genannt) besteht; und

einer Gruppe von Restriktionsenzym-Erkennungsstellen, welche aus KpnI-, SacII-, XbaI-, SalI-, ClaI-, PvuI- und StuI-Erkennungsstellen (nachstehend die Gruppe Nr. 4 von Erkennungsstellen genannt) besteht;
ausgewählt ist.

10. Verfahren nach Anspruch 9, welches das Einverleiben eines als Plasmid pHSG666 (FERM BP-1183) bezeichneten Plasmidvektors in den Mikroorganismus umfaßt.

11. Verfahren nach Anspruch 9, welches das Einverleiben eines als Plasmid pHSG671 (FERM BP-1184) bezeichneten Plasmidvektors in den Mikroorganismus umfaßt.

```
         Hpa I    Pvu II   Asu II              Sac I              Bal I
ATG.GCA.ACA.GTT.AAC.CAG.CTG.GTT.CGA.AAG.CCG.CGA.GCT.CGT.AAA.GTG.GCC.AAA.TCT.AAC.
Met-Ala-Thr-Val-Asn-Gln-Leu-Val-Arg-Lys-Pro-Arg-Ala-Arg-Lys-Val-Ala-Lys-Ser-Asn-

         Xho I   Sph I
GTT.CCG.GCT.CTC.GAG.GCA.TGC.CCG.CAA.AAA.CGT.GGC.GTA.TGT.ACT.CGT.GTA.TAT.ACT.ACC.
Val-Pro-Ala-Leu-Glu-Ala-Cys-Pro-Gln-Lys-Arg-Gly-Val-Cys-Thr-Arg-Val-Tyr-Thr-Thr-

ACT.CCT.AAA.AAA.CCG.AAC.TCC.GCG.CTG.CGT.AAA.GTA.TGC.CGT.GTT.CGT.CTG.ACT.AAC.GGT.
Thr-Pro-Lys-Lys-Pro-Asn-Ser-Ala-Leu-Arg-Lys-Val-Cys-Arg-Val-Arg-Leu-Thr-Asn-Gly-

                                                      Pst I
TTC.GAA.GTG.ACT.TCC.TAC.ATC.GGT.GGT.GAA.GGT.CAC.AAC.CTG.CAG.GAA.CAC.TCT.GTT.ATC.
Phe-Glu-Val-Thr-Ser-Tyr-Ile-Gly-Gly-Glu-Gly-His-Asn-Leu-Gln-Glu-His-Ser-Val-Ile-
  Bcl I   NotI Xma III            Bgl II   SmaI BamH I  Kpn I               Sac II
CTG.ATC.AGA.GGC.GGC.CGC.GTT.AAA.GAT.CTG.CCC.GGG.ATC.CGG.TAC.CAC.ACC.GTC.CGC.GGC.
Leu-Ile-Arg-Gly-Gly-Arg-Val-Lys-Asp-Leu-Pro-Gly-Ile-Arg-Tyr-His-Thr-Val-Arg-Gly-

   Xba I                                  Sal I  Cla I Pvu I
GCT.CTA.GAC.TGC.TCC.GGA.GTA.AAG.GAC.CGT.CGA.CAG.GAT.CGA.TCG.AAA.TAC.GGT.GTA.AAA.
Ala-Leu-Asp-Cys-Ser-Gly-Val-Lys-Asp-Arg-Arg-Gln-Asp-Arg-Ser-Lys-Tyr-Gly-Val-Lys-

         Stu I
CGT.CCG.AAG.GCC.TAA.
Arg-Pro-Lys-Ala ter"
```

**FIG.1**

EP 0 223 156 B1

```
        Hpa I  _Pvu II  __Asu II            Sac I           Bal I
ATG GCA ACA GTT AAC CAG CTG GTT CGA AAG CCG CGA GCT CGT AAA GTG GCC AAA TCT AAC
Met)Ala-Thr-Val-Asn-Gln-Leu-Val-Arg-Lys-Pro-Arg-Ala-Arg-Lys-Val-Ala-Lys-Ser-Asn


        XhoI  __SphI                           Mlu I __ Sna I
GTT CCG GCT CTC GAG GCA TGC CCG CAG AAG CGT GGT GTG TGC ACA CGC GTA TAC ACT ACT
Val-Pro-Ala-Leu-Glu-Ala-Cys-Pro-Gln-Lys-Arg-Gly-Val-Cys-Thr-Arg-Val-Tyr-Thr-Thr


        Eco RI          Mst I
ACT CCG AAG AAA CCG AAT TCT GCT CTG CGC AAA GTA TGC CGC GTA CGC CTG ACC AAC GGT
Thr-Pro-Lys-Lys-Pro-Asn-Ser-Ala-Leu-Arg-Lys-Val-Cys-Arg-Val-Arg-Leu-Thr-Asn-Gly


      Bst EII                                  Pst I
TTC GAG GTC ACC TCA TAT ATA GGT GGT GAA GGA CAC AAC CTG CAG GAA CAC TCT GTT ATC
Phe-Glu-Val-Thr-Ser-Tyr-Ile-Gly-Gly-Glu-Gly-His-Asn-Leu-Gln-Glu-His-Ser-Val-Ile

                Xma III
  Bcl I    NotI            Bgl II   Sma I  BamHI  Kpn I        SacII
CTG ATC AGA GGC GGC CGC GTT AAA GAT CTG CCC GGG ATC CGG TAC CAC ACC GTC CGC GGC
Leu-Ile-Arg-Gly-Gly-Arg-Val-Lys-Asp-Leu-Pro-Gly-Ile-Arg-Tyr-His-Thr-Val-Arg-Gly


  Xba I                           Sal I Cla I  Pvu I
GCT CTA GAC TGC TCC GGA GTA AAG GAC CGT CGA CAG GAT CGA TCG AAA TAC GGT GTA AAA
Ala-Leu-Asp-Cys-Ser-Gly-Val-Lys-Asp-Arg-Arg-Gln-Asp-Arg-Ser-Lys-Tyr-Gly-Val-Lys


      Stu I      Hind III
CGT CCG AAG GCC TAA TAG AAG CTT
Arg-Pro-Lys-Ala trm.trm.
```

# FIG. 2

FRAGMENTS  SYNTHESIZED                **FIG.3**

(A)   TCGACCATG GCAACAGTTA ACCAGCTGGT TCGAAAGCCG CGAGCT
              GGTAC CGTTGTCAAT TGGTCGACCA AGCTTTCGGC GC

(B)       CGTA AAGTGGCCAA ATCTAACGTT CCGGCTCTCG AGGCATG
        TCGAGCAT TTCACCGGTT TAGATTGCAA GGCCGAGAGC TCC

(C)       GATCTG CCCGGGGGTTC GGTACCACAC CGTCCGCGGC GCTCTAGACT GCTCCGGAGT-
                AC GGGCCCCAAG CCATGGTGTG GCAGGCGCCG CGAGATCTGA CGTGGCCTCA-

      -AAAGGACCG
      -TTTCCTGGCA GCT

(D)            T CGACAGGATC GATCGAAATA CGGTGTAAAA CGTCCGAAGG CCTAATAGA
                  GTCCTAG CTAGCTTTAT GCCACATTTT GCAGGCTTCC GGATTATCTT CGA

(E)       GGAACA CTCTGTTATC CTGATCAGAG GCGGCCGCGT TAAAGATCTG CCC
        ACGTCCTTGT GAGACAATAG GACTAGTCTC CGCCGGCGCA ATTTCTAGAC GGG

(F)       GATCTG CCCGGGGGATC CGGTAC
                AC GGGCCCCTAG GC

(G)           CCC GCAGAAGCGT GGTGTGTGCA CACGCGTATA CACTACTACT CCGAAGAAAC-
            GTACGGG CGTCTTCGCA CCACACACGT GTGCGCATAT GTGATGATGA GGCTTCTTTG-

        -CG
        -GCTTAA

(H)       AATTCAGC GCTGCGCAAG CTTTGCCGCG TACGCCTGAC CAACGGTTTC GAG
                GTCG CGACGCGTTC GAAACGGCGC ATGCGGACTG GTTGCCAAAG CTCCAGTG

(I)       GTCACCT CATATATAGG TGGTGAAGGA CACAACCTGC A
                GA GTATATATCC ACCACTTCCT GTGTTGG

(J)       AATTCTGC TCTGCGCAAA GTATGCCGCG TACGCCTGAC CAACGGTTTC GAG
                GACG AGACGCGTTT CATACGGCGC ATGCGGACTG GTTGCCAAAG CTCCAGTG

FIG. 4

FIG. 5

# FIG. 6

FIG.7

FIG. 8

EP 0 223 156 B1

# FIG. 9

FIG. 9a

1           10           20

pNO1523 Met-Ala-Thr-Val-Asn-Gln-Leu-Val-Arg-Lys-Pro-Arg-Ala-Arg-Lys-Val-Ala-Lys-Ser-Asn-
     ATG GCA ACA GTT AAC CAG CTG GTA CGA AAA CCA CGT GCT CGC AAA GTT GCG AAA AGC AAC
pHSG671 --- --- --- --- --- --- --- --T --- --G --G --A --- --T --- --G --C --- TCT ---
     Met-Ala-Thr-Val-Asn-Gln-Leu-Val-Arg-Lys-Pro-Arg-Ala-Arg-Lys-Val-Ala-Lys-Ser-Asn-

21           30           40

pNO1523 Val-Pro-Ala-Leu-Glu-Ala-Cys-Pro-Gln-Lys-Arg-Gly-Val-Cys-Thr-Arg-Val-Tyr-Thr-Thr-
     GTG CCT GCG CTG GAA GCA TGC CCG CAA AAA CGT GGC GTA TGT ACT CGT GTA TAT ACT ACC
pHSG671 --T --G --T --C --G --- --- --- --G --G --- --T --G --C --A --C --- --C --- --T
     Val-Pro-Ala-Leu-Glu-Ala-Cys-Pro-Gln-Lys-Arg-Gly-Val-Cys-Thr-Arg-Val-Tyr-Thr-Thr-

41           50           60

pNO1523 Thr-Pro-Lys-Lys-Pro-Asn-Ser-Ala-Leu-Arg-Lys-Val-Cys-Arg-Val-Arg-Leu-Thr-Asn-Gly-
     ACT CCT AAA AAA CCG AAC TCC GCG CTG CGT AAA GTA TGC CGT GTT CGT CTG ACT AAC GGT
pHSG671 --- --G --G --- --- --T --T --T --- --C --- --- --- --C --A --C --- --C --- ---
     Thr-Pro-Lys-Lys-Pro-Asn-Ser-Ala-Leu-Arg-Lys-Val-Cys-Arg-Val-Arg-Leu-Thr-Asn-Gly-

61           70           80

pNO1523 Phe-Glu-Val-Thr-Ser-Tyr-Ile-Gly-Gly-Glu-Gly-His-Asn-Leu-Gln-Glu-His-Ser-Val-Ile-
     TTC GAA GTG ACT TCC TAC ATC GGT GGT GAA GGT CAC AAC CTG CAG GAG CAC TCC GTG ATC
pHSG671 --- --G --C --C --A --T --A --- --- --- --A --- --- --- --- --A --- --T --T ---
     Phe-Glu-Val-Thr-Ser-Tyr-Ile-Gly-Gly-Glu-Gly-His-Asn-Leu-Gln-Glu-His-Ser-Val-Ile-

81           90           100

pNO1523 Leu-Ile-Arg-Gly-Gly-Arg-Val-Lys-Asp-Leu-Pro-Gly-Val-Arg-Tyr-His-Thr-Val-Arg-Gly-
     CTG ATC CGT GGC GGT CGT GTT AAA GAC CTC CCG GGT GTT CGT TAC CAC ACC GTA CGT GGC
pHSG671 --- --- A-A --- --C --C --- --- --T --G --C --G A-C --G --- --- --- --C --C ---
     Leu-Ile-Arg-Gly-Gly-Arg-Val-Lys-Asp-Leu-Pro-Gly-Ile-Arg-Tyr-His-Thr-Val-Arg-Gly-

101          110           120

pNO1523 Ala-Leu-Asp-Cys-Ser-Gly-Val-Lys-Asp-Arg-Lys-Gln-Ala-Arg-Ser-Lys-Tyr-Gly-Val-Lys-
     GCG CTT GAC TGC TCC GGC GTT AAA GAC CGT AAG CAG GCT CGT TCT AAG TAT GGC GTG AAG
pHSG671 --T --A --- --- --- --A --A --G --- --- CGA --- -A- --A --G --A --C --T --A --A
     Ala-Leu-Asp-Cys-Ser-Gly-Val-Lys-Asp-Arg-Arg-Gln-Asp-Arg-Ser-Lys-Tyr-Gly-Val-Lys-

121   124

pNO1523 Arg-Pro-Lys-Ala ter
     CGT CCT AAG GCT TAA
pHSG671 --- --G --- --C ---
     Arg-Pro-Lys-Ala ter

# FIG.10

FIG.11

FIG.12